# EUROPEAN PATENT APPLICATION

(11) **EP 4 052 712 A1**
(43) Date of publication of application: **07.09.2022**
(21) Application number: 21868273.0
(22) Date of filing: 26.07.2021
(51) Int. Cl.: A61K 31/715, A61K 31/702, A61P 31/14

(54) **SUGAR CHAIN AND COMPOSITIONS THEREOF AND USE THEREOF IN PREVENTION AND/OR TREATMENT OF CORONAVIRUS INFECTION**

(30) Priority: 18.09.2020 CN 202010987084
(71) Applicant: Institute Of Process Engineering Chinese Acadamy Of Sciences, 100190 Beijing (CN)
(72) Inventor: DU, Yuguang, Beijing 100190 (CN); LI, Jianjun, Beijing 100190 (CN); XU, Yueqiang, Beijing 100190 (CN); WANG, Zhuo, Beijing 100190 (CN); LIU, Dongdong, Beijing 100190 (CN); YAN, Yalu, Beijing 100190 (CN); WANG, Qi, Beijing 100190 (CN); YOU, Xin, Beijing 100190 (CN); DU, Xiaohui, Beijing 100190 (CN)
(74) Representative: karo IP
(86) International application number: PCT/CN2021/108344
(87) International publication number: WO 2022/057444

(57) **Abstract**

The present application belongs to the field of life health, and discloses a sugar chain and a composition thereof, and use in the prevention and/or treatment of coronavirus infection. The sugar chain contains any one or more of Neu5Acα2-N₁Gal building blocks, and/or any one or more of xFuc-N₁Gal-N₁(xFuc-N₁)GlcNAc building blocks, at the non-reducing end, where, x=0 or 1, and N₁=1, 2, 3, 4 or 6. A glycosidic bond formed between Neu5Ac and Gal is an α2 glycosidic bond. In the xFuc-N₁Gal-N₁(xFuc-N₁)GlcNAc building blocks, a glycosidic bond formed between any two adjacent monosaccharides is an α1 or β1 glycosidic bond. The specific building block contained at the non-reducing end of the sugar chain blocks the binding of the virus to the host, thereby blocking virus invasion and infection of the respiratory tract/lung, and achieving the specific prevention and treatment.

## Description

### Technical Field

The present application belongs to the field of life health, and relates to a sugar chain and a composition thereof, and use in the prevention and/or treatment of coronavirus infection.

### Background Art

The outbreak and epidemic of 2019 novel coronavirus (SARS-CoV-2) has posed a serious threat to human health, causing a heavy burden and huge losses to countries around the world. Studies have confirmed that SARS-Cov-2 is highly infectious and pathogenic, but there is still a lack of specific and effective drugs. How to effectively prevent SARS-Cov-2 spreading and infection is a key problem that needs to be solved urgently. SARS-CoV-2 invades the host by the binding of the S1 subunit of the S protein to three receptors, i.e. ACE2, DC-SIGN (C-type lectin on the surface of the dendritic cell), and L-SIGN (C-type lectin), of the host cell. Studies have proven that SARS-CoV-2 also infects the host by the binding of the S1 subunit to ACE2. In view of 66% sequence homology of the S1 subunits of SARS-CoV and SARS-CoV-2, SARS-CoV-2 may also infect the host by the binding of the S1 subunit to the other two receptors. Moreover, as the main symptom of SARS-CoV-2 infection of the host is infection of the lung, and ACE2, DC-SIGN, and L-SIGN are all expressed in the lung to a certain extent, the respiratory tract is considered to be one of the main ways of SARS-CoV-2 infection. Effectively blocking SARS-CoV-2 invasion of the respiratory tract of the host is an important strategy to prevent SARS-CoV-2 spreading and infection of the lung.

Heparin sulfate is a commonly used polysaccharide receptor for coronaviruses, belongs to linear polysaccharides, and usually binds to cell surface or extracellular matrix proteins to form glycoproteins so as to exert biological functions. The biological significance of the binding of the S proteins of coronaviruses to polysaccharides has attracted more and more attention. The S proteins of coronaviruses bind to saccharides mainly through the S1-NTD (N-terminal domain) functional domain. Recent studies have shown that the S1 subunit of SARS-CoV-2 can bind to heparin sulfate, and the degree of polymerization and sulfation are crucial to binding.

Human milk oligosaccharides (HMOs) form the third most abundant solid component of human milk after lactose and fat. Approximately 240 HMOs are known. Studies have shown that some HMOs have similar structures to ligands or receptors of pathogenic microorganisms on the surfaces of the intestinal tract and the respiratory tract, and can competitively bind to the pathogenic microorganisms, so that the pathogenic microorganisms cannot to adhere to the surfaces of the intestinal tract and the respiratory tract. Therefore, HMOs can reduce the incidence of intestinal and respiratory diseases. Studies have proven that some HMOs exhibit good anti-bacterial and anti-viral adhesion activities.

Studies have found that many human viruses, such as Middle East Respiratory Syndrome Coronavirus (MERS-CoV for short), can specifically bind to sugar chains containing sialic acid at the non-reducing end on the cell surface through the S1 subunit. However, recent studies have shown that the S1 subunit of SARS-CoV-2 cannot bind to sugar chains containing sialic acid at the non-reducing end.

Studies have confirmed that functional oligosaccharides, such as polygonatum oligosaccharide, carrageenan oligosaccharide, and chitosan oligosaccharide that are prepared from polysaccharides such as Chinese herbal medicine polysaccharides and seaweed polysaccharides, are safe, non-toxic, and water-soluble, and exhibit good anti-inflammatory, anti-bacterial, and anti-viral activities. Studies also have found that ginsenosides can inhibit excessive inflammation of macrophage NLRP3 inflammasome to protect against injury of organs, block ACE-2 enzyme required for virus insertion into lung cell receptors, and inhibit protease required for virus replication. Ginsenosides also have an anti-viral activity.

### Summary of the Invention

### 1. Problems to be solved

In view of the serious infectivity and pathogenicity of coronaviruses, there is still a lack of specific and effective drugs in the prior art, and it is difficult to achieve rapid prevention and treatment.

A first objective of the present application is to prepare a product for preventing and/or treating coronavirus infection from a single sugar chain or composition of multiple sugar chains containing a specific building block at the non-reducing end. The sugar chain specifically binds to the S1 subunit of the virus, and/or the DC-SIGN receptor of the host through the specific building block at the non-reducing end, thereby blocking the binding of the virus to the host receptor, blocking virus invasion and infection of the respiratory tract/lung, and achieving the specific prevention and treatment.

A second objective of the present application is to combine the sugar chain containing a specific building block at the non-reducing end with other sugar chains to form a complex sugar chain "cocktail". The complex sugar chain can bind to the S1 subunit of the virus and/or the DC-SIGN receptor of the host, and can also combine different methods for blocking the virus to achieve rapid and effect prevention and treatment by many different blocking methods.

### 2. Technical solutions

In order to solve the above problems, the present application adopts the following technical solutions.

The present application provides a sugar chain, which contains any one or more of Neu5Acα2-N₁Gal building blocks, and/or any one or more of xFuc-N₁Gal-N₁(xFuc-N₁)GlcNAc building blocks, at the non-reducing end, where, x=0 or 1, and N₁=1, 2, 3, 4 or 6.

Gal refers to galactose; Fuc refers to fucose; GlcNAc refers to N-acetylglucosamine; and Neu5Ac refers to sialic acid.

In some preferred embodiments, the Neu5Acα2-N₁Gal building blocks include Neu5Acα2-Gal, Neu5Acα2-2Gal, Neu5Acα2-3Gal, Neu5Acα2-4Gal, and Neu5Acα2-6Gal.

In some preferred embodiments, the xFuc-N₁Gal-N₁(xFuc-N₁)GlcNAc building blocks include N₁Gal-N₁GlcNAc, N₁Gal-N₁(Fuc-N₁)GlcNAc, Fuc-N₁Gal-N₁(Fuc-N₁)GlcNAc, and Fuc-N₁Gal-N₁GlcNAc.

In some preferred embodiments, the N₁Gal-N₁GlcNAc includes Gal-GlcNAc, Gal-2GlcNAc, Gal-3GlcNAc, Gal-4GlcNAc, Gal-6GlcNAc, 2Gal-GlcNAc, 2Gal-2GlcNAc, 2Gal-3GlcNAc, 2Gal-4GlcNAc, 2Gal-6GlcNAc, 3Gal-GlcNAc, 3Gal-2GlcNAc, 3Gal-3GlcNAc, 3Gal-4GlcNAc, 3Gal-6GlcNAc, 4Gal-GlcNAc, 4Gal-2GlcNAc, 4Gal-3GlcNAc, 4Gal-4GlcNAc, 4Gal-6GlcNAc, 6Gal-GlcNAc, 6Gal-2GlcNAc, 6Gal-3GlcNAc, 6Gal-4GlcNAc, and 6Gal-6GlcNAc.

In some preferred embodiments, the N₁Gal-N₁(Fuc-N₁)GlcNAc includes Gal-(Fuc-1)GlcNAc, Gal-2(Fuc-1)GlcNAc, Gal-3(Fuc-1)GlcNAc, Gal-4(Fuc-1)GlcNAc, Gal-6(Fuc-1)GlcNAc, 2Gal-(Fuc-1)GlcNAc, 2Gal-2(Fuc-1)GlcNAc, 2Gal-3(Fuc-1)GlcNAc, 2Gal-4(Fuc-1)GlcNAc, 2Gal-6(Fuc-1)GlcNAc, 3Gal-(Fuc-1)GlcNAc, 3Gal-2(Fuc-1)GlcNAc, 3Gal-3(Fuc-1)GlcNAc, 3Gal-4(Fuc-1)GlcNAc, 3Gal-6(Fuc-1)GlcNAc, 4Gal-(Fuc-1)GlcNAc, 4Gal-2(Fuc-1)GlcNAc, 4Gal-3(Fuc-1)GlcNAc, 4Gal-4(Fuc-1)GlcNAc, 4Gal-6(Fuc-1)GlcNAc, 6Gal-(Fuc-1)GlcNAc, 6Gal-2(Fuc-1)GlcNAc, 6Gal-3(Fuc-1)GlcNAc, 6Gal-4(Fuc-1)GlcNAc, and 6Gal-6(Fuc-1)GlcNAc;
Gal-(Fuc-2)GlcNAc, Gal-2(Fuc-2)GlcNAc, Gal-3(Fuc-2)GlcNAc, Gal-4(Fuc-2)GlcNAc, Gal-6(Fuc-2)GlcNAc, 2Gal-(Fuc-2)GlcNAc, 2Gal-2(Fuc-2)GlcNAc, 2Gal-3(Fuc-2)GlcNAc, 2Gal-4(Fuc-2)GlcNAc, 2Gal-6(Fuc-2)GlcNAc, 3Gal-(Fuc-2)GlcNAc, 3Gal-2(Fuc-2)GlcNAc, 3Gal-3(Fuc-2)GlcNAc, 3Gal-4(Fuc-2)GlcNAc, 3Gal-6(Fuc-2)GlcNAc, 4Gal-(Fuc-2)GlcNAc, 4Gal-2(Fuc-2)GlcNAc, 4Gal-3(Fuc-2)GlcNAc, 4Gal-4(Fuc-2)GlcNAc, 4Gal-6(Fuc-2)GlcNAc, 6Gal-(Fuc-2)GlcNAc, 6Gal-2(Fuc-2)GlcNAc, 6Gal-3(Fuc-2)GlcNAc, 6Gal-4(Fuc-2)GlcNAc, and 6Gal-6(Fuc-2)GlcNAc;
Gal-(Fuc-3)GlcNAc, Gal-2(Fuc-3)GlcNAc, Gal-3(Fuc-3)GlcNAc, Gal-4(Fuc-3)GlcNAc, Gal-6(Fuc-3)GlcNAc, 2Gal-(Fuc-3)GlcNAc, 2Gal-2(Fuc-3)GlcNAc, 2Gal-3(Fuc-3)GlcNAc, 2Gal-4(Fuc-3)GlcNAc, 2Gal-6(Fuc-3)GlcNAc, 3Gal-(Fuc-3)GlcNAc, 3Gal-2(Fuc-3)GlcNAc, 3Gal-3(Fuc-3)GlcNAc, 3Gal-4(Fuc-3)GlcNAc, 3Gal-6(Fuc-3)GlcNAc, 4Gal-(Fuc-3)GlcNAc, 4Gal-2(Fuc-3)GlcNAc, 4Gal-3(Fuc-3)GlcNAc, 4Gal-4(Fuc-3)GlcNAc, 4Gal-6(Fuc-3)GlcNAc, 6Gal-(Fuc-3)GlcNAc, 6Gal-2(Fuc-3)GlcNAc, 6Gal-3(Fuc-3)GlcNAc, 6Gal-4(Fuc-3)GlcNAc, and 6Gal-6(Fuc-3)GlcNAc;
Gal-(Fuc-4)GlcNAc, Gal-2(Fuc-4)GlcNAc, Gal-3(Fuc-4)GlcNAc, Gal-4(Fuc-4)GlcNAc, Gal-6(Fuc-4)GlcNAc, 2Gal-(Fuc-4)GlcNAc, 2Gal-2(Fuc-4)GlcNAc, 2Gal-3(Fuc-4)GlcNAc, 2Gal-4(Fuc-4)GlcNAc, 2Gal-6(Fuc-4)GlcNAc, 3Gal-(Fuc-4)GlcNAc, 3Gal-2(Fuc-4)GlcNAc, 3Gal-3(Fuc-4)GlcNAc, 3Gal-4(Fuc-4)GlcNAc, 3Gal-6(Fuc-4)GlcNAc, 4Gal-(Fuc-4)GlcNAc, 4Gal-2(Fuc-4)GlcNAc, 4Gal-3(Fuc-4)GlcNAc, 4Gal-4(Fuc-4)GlcNAc, 4Gal-6(Fuc-4)GlcNAc, 6Gal-(Fuc-4)GlcNAc, 6Gal-2(Fuc-4)GlcNAc, 6Gal-3(Fuc-4)GlcNAc, 6Gal-4(Fuc-4)GlcNAc, and 6Gal-6(Fuc-4)GlcNAc; and
Gal-(Fuc-6)GlcNAc, Gal-2(Fuc-6)GlcNAc, Gal-3(Fuc-6)GlcNAc, Gal-4(Fuc-6)GlcNAc, Gal-6(Fuc-6)GlcNAc, 2Gal-(Fuc-6)GlcNAc, 2Gal-2(Fuc-6)GlcNAc, 2Gal-3(Fuc-6)GlcNAc, 2Gal-4(Fuc-6)GlcNAc, 2Gal-6(Fuc-6)GlcNAc, 3Gal-(Fuc-6)GlcNAc, 3Gal-2(Fuc-6)GlcNAc, 3Gal-3(Fuc-6)GlcNAc, 3Gal-4(Fuc-6)GlcNAc, 3Gal-6(Fuc-6)GlcNAc, 4Gal-(Fuc-6)GlcNAc, 4Gal-2(Fuc-6)GlcNAc, 4Gal-3(Fuc-6)GlcNAc, 4Gal-4(Fuc-6)GlcNAc, 4Gal-6(Fuc-6)GlcNAc, 6Gal-(Fuc-6)GlcNAc, 6Gal-2(Fuc-6)GlcNAc, 6Gal-3(Fuc-6)GlcNAc, 6Gal-4(Fuc-6)GlcNAc, and 6Gal-6(Fuc-6)GlcNAc.

In some preferred embodiments, the Fuc-N₁Gal-N₁(Fuc-N₁)GlcNAc includes Fuc-Gal-(Fuc-1)GlcNAc, Fuc-Gal-2(Fuc-1)GlcNAc, Fuc-Gal-3 (Fuc-1)GlcNAc, Fuc-Gal-4(Fuc-1)GlcNAc, Fuc-Gal-6(Fuc-1)GlcNAc, Fuc-2Gal-(Fuc-1)GlcNAc, Fuc-2Gal-2(Fuc-1)GlcNAc, Fuc-2Gal-3 (Fuc-1)GlcNAc, Fuc-2Gal-4(Fuc-1)GlcNAc, Fuc-2Gal-6(Fuc-1)GlcNAc, Fuc-3 Gal-(Fuc-1)GlcNAc, Fuc-3 Gal-2(Fuc-1)GlcNAc, Fuc-3Gal-3(Fuc-1)GlcNAc, Fuc-3Gal-4(Fuc-1)GlcNAc, Fuc-3Gal-6(Fuc-1)GlcNAc, Fuc-4Gal-(Fuc-1)GlcNAc, Fuc-4Gal-2(Fuc-1)GlcNAc, Fuc-4Gal-3 (Fuc-1)GlcNAc, Fuc-4Gal-4(Fuc-1)GlcNAc, Fuc-4Gal-6(Fuc-1)GlcNAc, Fuc-6Gal-(Fuc-1)GlcNAc, Fuc-6Gal-2(Fuc-1)GlcNAc, Fuc-6Gal-3 (Fuc-1)GlcNAc, Fuc-6Gal-4(Fuc-1)GlcNAc, and Fuc-6Gal-6(Fuc-1)GlcNAc;
Fuc-Gal-(Fuc-2)GlcNAc, Fuc-Gal-2(Fuc-2)GlcNAc, Fuc-Gal-3(Fuc-2)GlcNAc, Fuc-Gal-4(Fuc-2)GlcNAc, Fuc-Gal-6(Fuc-2)GlcNAc, Fuc-2Gal-(Fuc-2)GlcNAc, Fuc-2Gal-2(Fuc-2)GlcNAc, Fuc-2Gal-3(Fuc-2)GlcNAc, Fuc-2Gal-4(Fuc-2)GlcNAc, Fuc-2Gal-6(Fuc-2)GlcNAc, Fuc-3Gal-(Fuc-2)GlcNAc, Fuc-3Gal-2(Fuc-2)GlcNAc, Fuc-3Gal-3(Fuc-1)GlcNAc, Fuc-3Gal-4(Fuc-2)GlcNAc, Fuc-3Gal-6(Fuc-2)GlcNAc, Fuc-4Gal-(Fuc-2)GlcNAc, Fuc-4Gal-2(Fuc-2)GlcNAc, Fuc-4Gal-3(Fuc-2)GlcNAc, Fuc-4Gal-4(Fuc-2)GlcNAc, Fuc-4Gal-6(Fuc-2)GlcNAc, Fuc-6Gal-(Fuc-2)GlcNAc, Fuc-6Gal-2(Fuc-2)GlcNAc, Fuc-6Gal-3(Fuc-2)GlcNAc, Fuc-6Gal-4(Fuc-2)GlcNAc, and Fuc-6Gal-6(Fuc-2)GlcNAc;
Fuc-Gal-(Fuc-3)GlcNAc, Fuc-Gal-2(Fuc-3)GlcNAc, Fuc-Gal-3(Fuc-3)GlcNAc, Fuc-Gal-4(Fuc-3)GlcNAc, Fuc-Gal-6(Fuc-3)GlcNAc, Fuc-2Gal-(Fuc-3)GlcNAc, Fuc-2Gal-2(Fuc-3)GlcNAc, Fuc-2Gal-3(Fuc-3)GlcNAc, Fuc-2Gal-4(Fuc-3)GlcNAc, Fuc-2Gal-6(Fuc-3)GlcNAc, Fuc-3Gal-(Fuc-3)GlcNAc, Fuc-3Gal-2(Fuc-3)GlcNAc, Fuc-3Gal-3(Fuc-1)GlcNAc, Fuc-3Gal-4(Fuc-3)GlcNAc, Fuc-3Gal-6(Fuc-3)GlcNAc, Fuc-4Gal-(Fuc-3)GlcNAc, Fuc-4Gal-2(Fuc-3)GlcNAc, Fuc-4Gal-3(Fuc-3)GlcNAc, Fuc-4Gal-4(Fuc-3)GlcNAc, Fuc-4Gal-6(Fuc-3)GlcNAc, Fuc-6Gal-(Fuc-3)GlcNAc, Fuc-6Gal-2(Fuc-3)GlcNAc, Fuc-6Gal-3(Fuc-3)GlcNAc, Fuc-6Gal-4(Fuc-3)GlcNAc, and Fuc-6Gal-6(Fuc-3)GlcNAc;
Fuc-Gal-(Fuc-4)GlcNAc, Fuc-Gal-2(Fuc-4)GlcNAc, Fuc-Gal-3(Fuc-4)GlcNAc, Fuc-Gal-4(Fuc-4)GlcNAc, Fuc-Gal-6(Fuc-4)GlcNAc, Fuc-2Gal-(Fuc-4)GlcNAc, Fuc-2Gal-2(Fuc-4)GlcNAc, Fuc-2Gal-3(Fuc-4)GlcNAc, Fuc-2Gal-4(Fuc-4)GlcNAc, Fuc-2Gal-6(Fuc-4)GlcNAc, Fuc-3Gal-(Fuc-4)GlcNAc, Fuc-3Gal-2(Fuc-4)GlcNAc, Fuc-3Gal-3(Fuc-4)GlcNAc, Fuc-3Gal-4(Fuc-4)GlcNAc, Fuc-3Gal-6(Fuc-4)GlcNAc, Fuc-4Gal-(Fuc-4)GlcNAc, Fuc-4Gal-2(Fuc-4)GlcNAc, Fuc-4Gal-3(Fuc-4)GlcNAc, Fuc-4Gal-4(Fuc-4)GlcNAc, Fuc-4Gal-6(Fuc-4)GlcNAc, Fuc-6Gal-(Fuc-4)GlcNAc, Fuc-6Gal-2(Fuc-4)GlcNAc, Fuc-6Gal-3(Fuc-4)GlcNAc, Fuc-6Gal-4(Fuc-4)GlcNAc, and Fuc-6Gal-6(Fuc-4)GlcNAc; and
Fuc-Gal-(Fuc-6)GlcNAc, Fuc-Gal-2(Fuc-6)GlcNAc, Fuc-Gal-3(Fuc-6)GlcNAc, Fuc-Gal-4(Fuc-6)GlcNAc, Fuc-Gal-6(Fuc-6)GlcNAc, Fuc-2Gal-(Fuc-6)GlcNAc, Fuc-2Gal-2(Fuc-6)GlcNAc, Fuc-2Gal-3(Fuc-6)GlcNAc, Fuc-2Gal-4(Fuc-6)GlcNAc, Fuc-2Gal-6(Fuc-6)GlcNAc, Fuc-3Gal-(Fuc-6)GlcNAc, Fuc-3Gal-2(Fuc-6)GlcNAc, Fuc-3Gal-3(Fuc-6)GlcNAc, Fuc-3Gal-4(Fuc-6)GlcNAc, Fuc-3Gal-6(Fuc-6)GlcNAc, Fuc-4Gal-(Fuc-6)GlcNAc, Fuc-4Gal-2(Fuc-6)GlcNAc, Fuc-4Gal-3(Fuc-6)GlcNAc, Fuc-4Gal-4(Fuc-6)GlcNAc, Fuc-4Gal-6(Fuc-6)GlcNAc, Fuc-6Gal-(Fuc-6)GlcNAc, Fuc-6Gal-2(Fuc-6)GlcNAc, Fuc-6Gal-3(Fuc-6)GlcNAc, Fuc-6Gal-4(Fuc-6)GlcNAc, and Fuc-6Gal-6(Fuc-6)GlcNAc.

In some preferred embodiments, the Fuc-N₁Gal-N₁GlcNAc includes Fuc-Gal-GlcNAc, Fuc-Gal-2GlcNAc, Fuc-Gal-3GlcNAc, Fuc-Gal-4GlcNAc, Fuc-Gal-6GlcNAc, Fuc-2Gal-GlcNAc, Fuc-2Gal-2GlcNAc, Fuc-2Gal-3GlcNAc, Fuc-2Gal-4GlcNAc, Fuc-2Gal-6GlcNAc, Fuc-3Gal-GlcNAc, Fuc-3Gal-2GlcNAc, Fuc-3Gal-3GlcNAc, Fuc-3Gal-4GlcNAc, Fuc-3Gal-6GlcNAc, Fuc-4Gal-GlcNAc, Fuc-4Gal-2GlcNAc, Fuc-4Gal-3GlcNAc, Fuc-4Gal-4GlcNAc, Fuc-4Gal-6GlcNAc, Fuc-6Gal-GlcNAc, Fuc-6Gal-2GlcNAc, Fuc-6Gal-3GlcNAc, Fuc-6Gal-4GlcNAc, and Fuc-6Gal-6GlcNAc.

In some preferred embodiments, in the Neu5Ac-Gal building blocks, a glycosidic bond formed between Neu5Ac and Gal is an α2 glycosidic bond; and in the xFuc-N₁Gal-N₁(xFuc-N₁)GlcNAc building blocks, a glycosidic bond formed between any two adjacent monosaccharides is an α1 or β1 glycosidic bond.

In some preferred embodiments, in the xFuc-N₁Gal-N₁(xFuc-N₁)GlcNAc building blocks, a glycosidic bond formed between Fuc and Gal is an α1 or β1 glycosidic bond, a glycosidic bond formed between Gal and GlcNAc is an α1 or β1 glycosidic bond, and a glycosidic bond formed between Fuc and GlcNAc is an α1 or β1 glycosidic bond.

α1 (alpha1) refers to that the hydroxyl at position C1 of a monosaccharide forms a glycosidic bond with an adjacent monosaccharide in an α (alpha) configuration; and β1 (beta1) refers to that the hydroxyl at position C1 of a monosaccharide forms a glycosidic bond with an adjacent monosaccharide in a β (beta) configuration.

For a monosaccharide that is not sialic acid, the hydroxyl at position C1 of the monosaccharide can form a glycosidic bond with an adjacent monosaccharide in an α (alpha) or β (beta) configuration; and for a monosaccharide that is sialic acid, the hydroxyl at position C2 of sialic acid can form a glycosidic bond with an adjacent monosaccharide in an α (alpha) configuration, i.e. α2.

For the xFuc-N₁Gal-N₁(xFuc-N₁)GlcNAc building blocks with brackets, a monosaccharide in the brackets means that the monosaccharide forms a branched chain, if the monosaccharide is sialic acid, the hydroxyl at position C2 of sialic acid forms a glycosidic bond with an adjacent monosaccharide in an α (alpha) configuration, and if the monosaccharide is not sialic acid, the hydroxyl at position C1 of the monosaccharide forms a glycosidic bond with an adjacent monosaccharide in an α (alpha) or β (beta) configuration.

In some preferred embodiments, the xFuc-N₁Gal-N₁(xFuc-N₁)GlcNAc building blocks include xFuc-N₁Gal-(xFuc-N₁)GlcNAcα1 or xFuc-N₁Gal-(xFuc-N₁)GlcNAcβ1, where, x=0 or 1, and N₁=1, 2, 3, 4, or 6. A glycosidic bond formed between GlcNAc and the following monosaccharide is an α1 or β1 glycosidic bond.

In some preferred embodiments, the xFuc-N₁Gal-N₁(xFuc-N₁)GlcNAc building blocks include xFucα1-N₁Galβ1-N₁(xFucα1-N₁)GlcNAcβ1, where, x=0 or 1, and N₁=1, 2, 3, 4 or 6.

In some preferred embodiments, the Neu5Ac-Gal building blocks include the Neu5Acα2-6Gal or Neu5Acα2-3Gal building block, and/or the xFuc-Gal-(xFuc-N₁)GlcNAc building blocks include the Lewis a, Lewis x, Lewis y or Blood Group H building block.

In some preferred embodiments, the sugar chain contains A and B building blocks at the non-reducing end, the A building block is any one or more of Lewis a, Lewis x, Lewis y, and Blood Group H, and the B building block is the Neu5Acα2-6Gal and/or Neu5Acα2-3Gal building blocks.

In some preferred embodiments, the sugar chain contains a combination of at least two of the Lewis a, Lewis x, Lewis y, and Blood Group H building blocks at the non-reducing end.

The structural formula of Neu5Acα2-6Gal is:

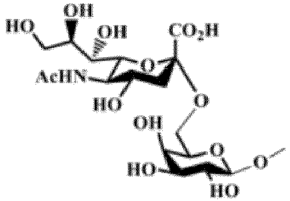

The structural formula of Neu5Acα2-3Gal is:

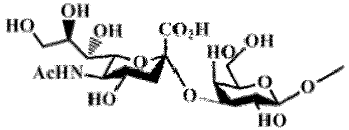

The structural formula of Lewis a is:

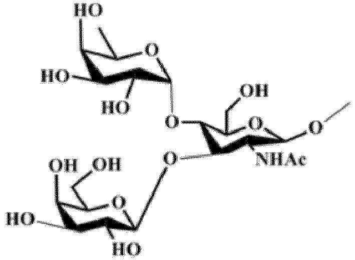

The structural formula of Lewis x is:

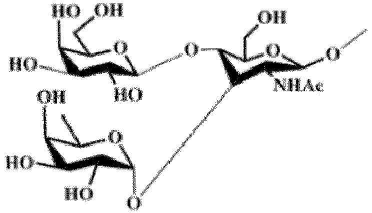

The structural formula of Lewis y is:

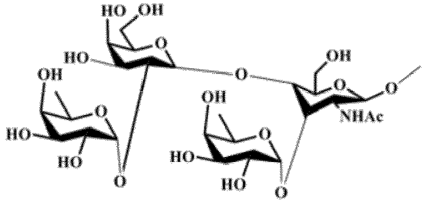

The structural formula of Blood Group H type II is:

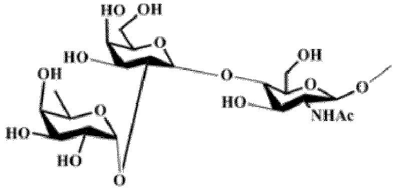

The above building blocks can also be expressed in the following manner:
Neu5Acα2-6Gal;
Neu5Acα2-3Gal;
Lewis a: Galβ1-3(Fucα1-4)GlcNAcβ;
Lewis y: Fucα1-2Galβ1-4(Fucα1-3)GlcNAcβ;
Lewis x: Galβ1-4(Fucα1-3)GlcNAcβ; and
Blood Group H type II: Fucα1-2Galβ1-4GlcNAcβ.

In some preferred embodiments, the sugar chain is any one of Fucα1-2Galβ1-4(Fucα1-3)GlcNAcβ1-3Galβ1-3GalNAcα, Fucα1-2Galβ1-4(Fucα1-3)GlcNAcβ1-3GalNAcα, Neu5Acα2-3Galβ1-4GlcNAcβ1-3GalNAcα, Neu5Acα2-6Galβ1-4GlcNAcβ1-3(Neu5Acα2-6Galβ1-3GlcNAcβ1-6)GalNAcα, Galβ1-4(Fucα1-3)GlcNAcβ1-3Galβ1-3GalNAcα, Galβ1-4(Fucα1-3)GlcNAcβ1-3GalNAcα, Fucα1-2Galβ1-4(Fucα1-3)GlcNAcβ1-6GalNAcα, 3 '-sialyllactose, 3 '-sialyllactosamine, 6'-sialyllactose, 6'-*N*-acetyl sialyllactosamine, Galβ1-3(Fucα1-4)GlcNAcβ1-3Galβ1-4Glc, Galβ1-4(Fucα1-3)GlcNAcβ1-3Galβ1-4Glc, Fucα1-2Galβ1-4GlcNAcβ1-3Galβ1-4Glc, Fucα1-2Galβ1-4(Fucα1-3)GlcNAcβ1-3Galβ1-4Glc, Galβ1-4(Fucα1-3)GlcNAcβ1-6(GlcNAcβ1-3)Galβ1-4Glc, Galβ1-4(Fucα1-3)GlcNAcβ1-6(Galβ1-4GlcNAcβ1-3)Galβ1-4Glc, Fuca1-2Galβ1-4GlcNAcβ1-6(Fucα1-2Galβ1-4GlcNAcβ1-3)Galβ1-4Glc, and Fucα1-2Galβ1-4(Fucα1-3)GlcNAcβ1-6(Fucα1-2Galβ1-4(Fucα1-3)GlcNAcβ1-3)Galβ1-4Glc.

Among the above sugar chains, Neu5Acα2-6Galβ1-4GlcNAcβ1-3(Neu5Acα2-6Galβ1-3GlcNAcβ1-6)GalNAc, 6'-sialyllactose (Neu5Acα2-6Galβ1-4Glc), and 6'-*N*-acetyl sialyllactosamine (Neu5Acα2-6Galβ1-4GlcNAc) are sugar chains containing the Neu5Acα2-6Gal building block at the non-reducing end.

Among the above sugar chains, Neu5Acα2-3Galβ1-4GlcNAcβ1-3GalNAcα, 3'-sialyllactose (Neu5Acα2-3Galβ1-4Glc), and 3'-sialyllactosamine (Neu5Acα2-3Galβ1-4GlcNAc) are sugar chains containing the Neu5Acα2-3Gal building block at the non-reducing end.

Among the above sugar chains, Galβ1-3(Fucα1-4)GlcNAcβ1-3Galβ1-4Glc is a sugar chain containing the Lewis a (Galβ1-3(Fucα1-4)GlcNAcβ-) building block at the non-reducing end.

Among the above sugar chains, Galβ1-4(Fucα1-3)GlcNAcβ1-3Galβ1-3GalNAcα, Galβ1-4(Fucα1-3)GlcNAcβ1-3GalNAcα, Galβ1-4(Fucα1-3)GlcNAcβ1-3Galβ1-4Glc, Galβ1-4(Fucα1-3)GlcNAcβ1-6(GlcNAcβ1-3)Galβ1-4Glc, and Galβ1-4(Fucα1-3)GlcNAcβ1-6(Galβ1-4GlcNAcβ1-3)Galβ1-4Glc are sugar chains containing the Lewis x (Galβ1-4(Fucα1-3)GlcNAcβ-) building block at the non-reducing end.

Among the above sugar chains, Fucα1-2Galβ1-4(Fucα1-3)GlcNAcβ1-3Galβ1-3GalNAcα, Fuca1-2Galβ1-4(Fucα1-3)GlcNAcβ1-3GalNAcα, Fucα1-2Galβ1-4(Fucα1-3)GlcNAcβ1-6GalNAcα, Fucα1-2Galβ1-4(Fucα1-3)GlcNAcβ1-3Galβ1-4Glc, and Fucα1-2Galβ1-4(Fucα1-3)GlcNAcβ1-6(Fucα1-2Galβ1-4(Fucα1-3)GlcNAcβ1-3)Galβ1-4Glc are sugar chains containing the Lewis y (Fucα1-2Galβ1-4(Fucα1-3)GlcNAcβ-) building block at the non-reducing end.

Among the above sugar chains, Fucα1-2Galβ1-4GlcNAcβ1-3Galβ1-4Glc and Fuca1-2Galβ1-4GlcNAcβ1-6(Fucα1-2Galβ1-4GlcNAcβ1-3)Galβ1-4Glc are sugar chains containing the Blood Group H (Fucα1-2Galβ1-4GlcNAcβ-) building block at the non-reducing end.

In some preferred embodiments, the present application provides a complex sugar chain, which is a sugar chain composition containing at least two different sugar chains. The sugar chain contains any one or more of Neu5Acα2-N₁Gal building blocks, and/or any one or more of xFuc-N₁Gal-N₁ (xFuc-N₁) GlcNAc building blocks, at the non-reducing end, where, x=0 or 1, and N₁=1, 2, 3, 4 or 6.

In some preferred embodiments, the complex sugar chain is a sugar chain composition containing at least two different sugar chains, and at least one sugar chain in the sugar chain composition contains any one or more of the following building blocks at the non-reducing end:
a) Neu5Acα2-6Gal;
b) Neu5Acα2-3Gal;
d) Lewis a;
e) Lewis x;
f) Lewis y; and
g) Blood Group H.

In some preferred embodiments, at least one sugar chain in the sugar chain composition is any one of sulfated fucoidan, HMOs, ginsenosides, chitosan oligosaccharide, heparin sulfate, heparin sulfate oligosaccharide, chondroitin sulfate, chondroitin sulfate oligosaccharide, dermatan sulfate, keratan sulfate, hyaluronic acid, hyaluronic acid oligosaccharide, fucoidan, fucose oligosaccharide, protein N/O-sugar chains, and exopolysaccharide from *Haloarcula hispanica.*

In the embodiments of the present application, the S1 protein of SARS-CoV-2 can bind to heparin sulfate, chondroitin sulfate, exopolysaccharides, fucoidan, 3-fucosyllactose, 2'-fucosyllactose, and sulfated fucoidan, respectively. Therefore, addition of the above oligosaccharides to the sugar chain composition can effectively block the binding of the S1 protein of SARS-CoV-2 to receptors.

In some embodiments, the S1 subunit of SARS-CoV-2 can also bind to ginsenosides, chitosan oligosaccharide, hyaluronic acid or protein N/O-sugar chains to block the binding of the S1 protein of SARS-CoV-2 to receptors.

In some preferred embodiments, each sugar chain in the sugar chain composition independently contains any one or more of the following building blocks at the non-reducing end:
a) Neu5Acα2-6Gal;
b) Neu5Acα2-3Gal;
d) Lewis a;
e) Lewis x;
f) Lewis y; and
g) Blood Group H.

When used in combination, the sugar chains containing the above building blocks can cooperate with each other to better block the binding of the virus to receptors.

In some preferred embodiments, at least one sugar chain in the sugar chain composition is any one of GlcNAcβ1-6(Galβ1-3)GalNAcα, GlcNAcβ1-6[Galβ1-4(Fucα1-3)GlcNAcβ1-3]Galβ1-4Glc, GlcNAcβ1-6[Fucα1-2Galβ1-4(Fucα1-3)GlcNAcβ1-3]Galβ1-4Glc, Galβ1-4GlcNAcβ1-6(Fucα1-2Galβ1-4GlcNAcβ1-3)Galβ1-4Glc, Galβ1-4(Fucα1-3)Glc, Fucα1-2Galβ1-4Glc, Galα1-3(Fucα1-2)Galβ1-4GlcNAcβ1-3NAcα, GlcNAcβ1-6(GlcNAcβ1-3)GalNAcα, Galβ1-4(Fucα1-6)GlcNAcβ1-3GalNAcα, and GlcNAcβ1-6(GlcNAcβ1-3)GalNAcα.

In some preferred embodiments, at least one sugar chain in the sugar chain composition is exopolysaccharide from *Haloarcula hispanica.*

In some preferred embodiments, in the sugar chain composition, the two sugar chains Fucα1-2Galβ1-4(Fucα1-3)GlcNAcβ1-3Galβ1-3GalNAcα and Fucα1-2Galβ1-4(Fucα1-3)GlcNAcβ1-3GalNAcα respectively containing the Lewis y building block can bind to not only the S1 subunit of the virus, but also the DC-SIGN receptor, so the sugar chain composition can prevent the virus from entering the host by two methods for blocking the virus.

In some preferred embodiments, the sugar chain composition contains at least one of Fucα1-2Galβ1-4(Fucα1-3)GlcNAcβ1-3Galβ1-3GalNAcα, and/or Fucα1-2Galβ1-4(Fucα1-3)GlcNAcβ1-3GalNAcα, and/or Neu5Acα2-3Galβ1-4GlcNAcβ1-3GalNAcα, and/or Neu5Acα2-6Galβ1-4GlcNAcβ1-3(Neu5Acα2-6Galp1-3GlcNAcβ1-6)GalNAcα, and/or Galβ1-4(Fucα1-3)GlcNAcβ1-3Galβ1-3GalNAcα, and/or Galβ1-4(Fucα1-3)GlcNAcβ1-3GalNAcα, and/or Fucα1-2Galβ1-4(Fucα1-3)GlcNAcβ1-6GalNAcα, and/or 3'-sialyllactose, and/or 3'-sialyllactosamine, and/or 6'-sialyllactose, and/or 6'-*N*-acetyl sialyllactosamine, and/or Galβ1-3(Fucα1-4)GlcNAcβ1-3Galβ1-4Glc, and/or Galβ1-4(Fucα1-3)GlcNAcβ1-3Galβ1-4Glc, and/or Fucα1-2Galβ1-4GlcNAcβ1-3Galβ1-4Glc, and/or Fucα1-2Galβ1-4(Fucα1-3)GlcNAcβ1-3Galβ1-4Glc, and/or Galβ1-4(Fucα1-3)GlcNAcβ1-6(GlcNAcβ1-3)Galβ1-4Glc, and/or Galβ1-4(Fucα1-3)GlcNAcβ1-6(Galβ1-4GlcNAcβ1-3)Galβ1-4Glc, and/or Fucα1-2Galβ1-4GlcNAcβ1-6(Fucα1-2Galβ1-4GlcNAcβ1-3)Galβ1-4Glc, and/or Fucα1-2Galβ1-4(Fucα1-3)GlcNAcβ1-6(Fucα1-2Galβ1-4(Fucα1-3)GlcNAcβ1-3)Galβ1-4Glc.

In some preferred embodiments, the present application provides use of the sugar chain and the complex sugar chain in the preparation of a composition for preventing and/or treating coronavirus infection.

In some preferred embodiments, the coronaviruses include coronaviruses containing the S1 subunit, and/or coronaviruses capable of binding to the DC-SIGN receptor.

In some preferred embodiments, the viruses include 2019-nCoV, MERS-CoV, SARS, and SARS-like viruses.

In some preferred embodiments, the present application provides use of a sugar chain in the preparation of a composition for preventing and/or treating coronavirus infection.

In some preferred embodiments, the present application provides use of exopolysaccharide from *Haloarcula hispanica* in the preparation of a composition for preventing and/or treating coronavirus infection.

In some preferred embodiments, an active ingredient of the composition contains the sugar chain or the complex sugar chain.

The composition of the present application contains a therapeutically effective amount of sugar chain, and one or more pharmaceutically acceptable carriers.

"Pharmaceutically acceptable carrier" refers to one or more compatible solids or liquid fillers or gelatinous substances, which are suitable for human use, and must have sufficient purity and low enough toxicity.

Some examples of the pharmaceutically acceptable carrier include cellulose and its derivatives (e.g. sodium carboxymethyl cellulose, sodium ethyl cellulose, and cellulose acetate), gelatin, talc, solid lubricants (e.g. stearic acid and magnesium stearate), calcium sulfate, plant oils (e.g. soybean oil, sesame oil, peanut oil, and olive oil), polyols (e.g. propylene glycol, glycerol, mannitol, and sorbitol), emulsifiers, wetting agents (e.g. sodium dodecyl sulfate), colorants, flavouring agents, stabilizers, antioxidants, preservatives, pyrogen-free water, etc.

The composition of the present application can be in a variety of forms, which may be any one selected from powder, ointment, paste, emulsion, gel, solution, patch, and inhalant. The composition can be administrated orally or parenterally in the form of topical administration. The sugar chain and the pharmaceutical composition of the present application can be loaded into a suitable solid or liquid carrier and into a suitable sterile device for injection or instillation. The above formulations can be prepared by conventional pharmaceutical methods.

The sugar chain or the composition of the present application can be clinically used for mammals including humans and animals, and can be administrated by oral, nasal or pulmonary route.

In some preferred embodiments, the pharmaceutical composition is used for topical administration.

In some preferred embodiments, the pharmaceutical composition is used for mucosal administration.

In some preferred embodiments, the administration methods include any one of oral gargle, nasal rinse, and gasifying agent-assisted delivery to the lung.

In some preferred embodiments, the present application provides a drug for preventing and/or treating coronavirus infection.

In some preferred embodiments, the present application provides a daily product for preventing and/or treating coronavirus infection.

In some preferred embodiments, the drug is in any form selected from powder, ointment, paste, emulsion, gel, solution, patch, and inhalant.

In some preferred embodiments, the daily product for preventing and/or treating coronavirus infection is used in the form of mouthwash or oral spray.

In some preferred embodiments, the active concentration of a sugar chain in the daily product for preventing and/or treating coronavirus infection is 0.01%-1% (W/V).

In some preferred embodiments, the present application provides use of a sugar chain or complex sugar chain in the preparation of a saccharide chip for preventing and/or treating coronavirus infection.

In some preferred embodiments, the present application provides a saccharide chip for preventing and/or treating coronavirus infection, which includes the sugar chain or complex sugar chain.

### 3. Beneficial effects

Compared with the prior art, the present application has the following beneficial effects.
(1) According to the present application, a product for preventing and/or treating coronavirus infection is prepared from a sugar chain containing a specific building block at the non-reducing end. The sugar chain specifically binds to the S 1 subunit of the virus, and/or the DC-SIGN receptor of the host through the specific building block at the non-reducing end, thereby blocking the binding of the virus to the host receptor, blocking virus invasion and infection of the respiratory tract/lung, and achieving the specific prevention and treatment.
(2) According to the present application, a complex sugar chain is formed by combining multiple sugar chains containing different functional building blocks at the non-reducing end, so the complex sugar chain can block virus invasion by many methods. A sugar chain containing the Neu5Acα2-6Gal and/or Neu5Acα2-3Gal building blocks at the non-reducing end is combined with a sugar chain containing the Lewis a, and/or Lewis x, and/or Lewis y, and/or Blood Group H building blocks at the non-reducing end. On the one hand, the functional sugar chains can directly bind to the S1 subunit of the virus to block the binding of the S1 subunit of the coronavirus to the lung of the infected patient. On the other hand, the functional sugar chains can specifically bind to the DC-SIGN receptor of the host to block virus invasion. Thus, the binding of the virus to the receptor is fundamentally blocked by different methods, virus invasion of organs is strictly blocked, and an effect of the specific prevention or treatment is significantly improved.
(3) According to the present application, the complex sugar chain is combined with a sugar chain capable of effectively blocking the binding of the S1 protein of the coronavirus to receptors, such as heparin sulfate, and/or chondroitin sulfate, and/or exopolysaccharides, and/or fucoidan, and/or a combination of 3-fucosyllactose, 2'-fucosyllactose, and sulfated fucoidan. Addition of the sugar chain to the complex sugar chain can obtain a variety of methods for blocking the virus, and a complex sugar chain "lung mask" capable of effectively preventing the virus from invading via the lung and the respiratory tract can be obtained.

### Brief Description of the Drawings

Figure 1 is a diagram of structural formulas of Neu5Acα2-6Gal, Neu5Acα2-3Gal, Lewis a, Lewis x, Lewis y, and Blood Group H type II, and in the figure, 1A shows Neu5Acα2-6Gal; 1B shows Neu5Acα2-3Gal; 1C shows Lewis a; 1D shows Lewis x; 1E shows Lewis y; and 1F shows Blood Group H type II;
Figure 2 is a ¹H NMR image of 6'-sialyllactose obtained by enzymatic synthesis of Example 1;
Figure 3 is a ¹³C NMR image of 6'-sialyllactose obtained by enzymatic synthesis of Example 1;
Figure 4 is a mass spectrogram of 6'-sialyllactose obtained by enzymatic synthesis of Example 1;

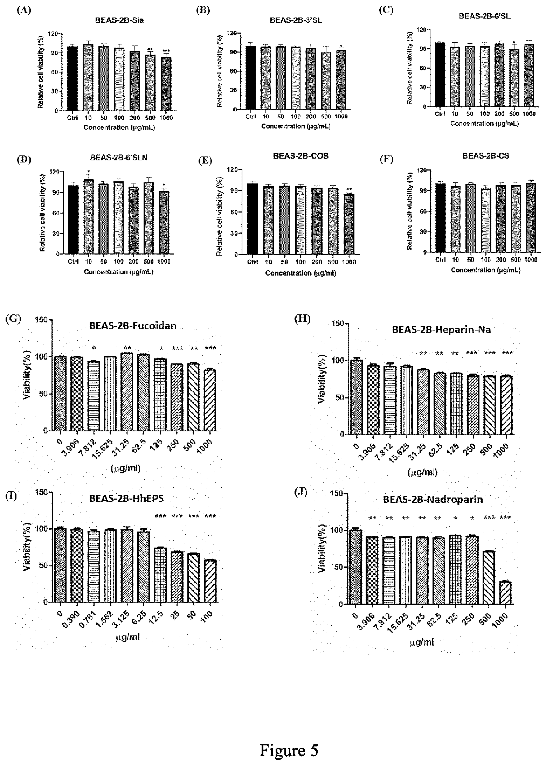

Figure 5 is a diagram of the toxicity of sialic acid, sialyloligosaccharides, chitosan oligosaccharide, and chondroitin sulfate of Example 2 to lung epithelial cells (BEAS-2B cells), and the in the figure, A shows sialic acid (Sia); B shows 3'-sialyllactose (3'-SL); C shows 6'-sialyllactose (6'-SL); D shows 6'-*N*-acetyl sialyllactosamine (6'-SLN); E shows chitosan oligosaccharide (COS); F shows chondroitin sulfate (CS); G shows fucoidan (Fucoidan); H shows sodium heparin sulfate (Heparin-Na); I shows exopolysaccharide from *Haloarcula hispanica* (HhEPS); and J shows nadroparin calcium (Nadroparin);
Figure 6 is a diagram of the toxicity of sialic acid, sialyloligosaccharides, chitosan oligosaccharide, and chondroitin sulfate of Example 2 to bronchial epithelial cells (16HBE cells), and the in the figure, A shows sialic acid (Sia); B shows 3'-sialyllactose (3'-SL); C shows 6'-sialyllactose (6'-SL); D shows 6'-*N*-acetyl sialyllactosamine (6'-SLN); E shows chitosan oligosaccharide (COS); and F shows chondroitin sulfate (CS);
Figure 7 is a diagram of the toxicity of fucoidan, nadroparin calcium, chitosan oligosaccharide, and exopolysaccharide from *Haloarcula hispanica* of Example 2 to African green monkey kidney cells (Vero E6 cells), and the in the figure, A shows fucoidan (Fucoidan); B shows nadroparin calcium (Nadroparin); and C shows exopolysaccharide from *Haloarcula hispanica* (HhEPS);
Figure 8 is a diagram of the binding of the S1 subunit of a virus to an O-sugar chain chip in an experiment of Example 3;
Figure 9 is a diagram of the binding of the DC-SIGN receptor to an O-sugar chain chip in an experiment of Example 3;
Figure 10 is a diagram of the binding of the DC-SIGN receptor to an HMO chip in an experiment of Example 3;
Figure 11 is a diagram of the binding of the S1 subunit of a virus to heparin sulfate in an experiment of Example 4;
Figure 12 is a diagram of the binding of the S1 subunit of a virus to chondroitin sulfate in an experiment of Example 4;
Figure 13 is a diagram of the binding of the S1 subunit of a virus to exopolysaccharide from *Haloarcula hispanica* in an experiment of Example 4;
Figure 14 is a diagram of the binding of the S1 subunit of a virus to fucoidan in an experiment of Example 4;
Figure 15 is a diagram of the binding of the S1 subunit of a virus to 3-fucosyllactose in an experiment of Example 4;
Figure 16 is a diagram of the binding of the S1 subunit of a virus to 2'-fucosyllactose in an experiment of Example 4;
Figure 17 is a diagram of the binding of the S1 subunit of a virus to nadroparin calcium in an experiment of Example 4;
Figure 18 is a diagram of an observation result of a control without any sugar chain of Example 5;
Figure 19 is a diagram of blocking the binding of the S1 subunit to 16HBE cells (bronchial epithelial cells) by chondroitin sulfate;
Figure 20 is a diagram of blocking the binding of the S1 subunit to 16HBE cells (bronchial epithelial cells) by fucoidan;

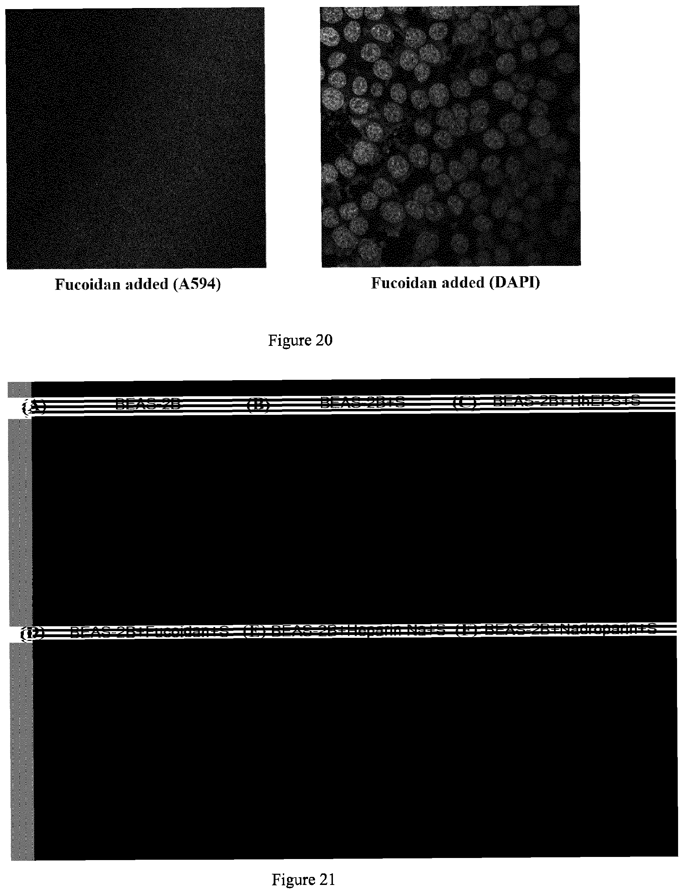

Figure 21 is a diagram of blocking the S1 subunit (the S1 protein) of SARS-CoV-2 to lung epithelial cells (BEAS-2B cells) by sugar chains of Example 6, and in the figure, A shows BEAS-2B cells; B shows the binding of the S1 protein to BEAS-2B cells; C shows an inhibitory effect of exopolysaccharide from *Haloarcula hispanica* (HhEPS) on the binding of the S1 protein to BEAS-2B cells; D shows an inhibitory effect of fucoidan (Fucoidan) on the binding of the S1 protein to BEAS-2B cells; E shows an inhibitory effect of sodium heparin sulfate (Heparin-Na) on the binding of the S1 protein to BEAS-2B cells; and F shows an inhibitory effect of nadroparin calcium (Nadroparin) on the binding of the S1 protein to BEAS-2B cells;

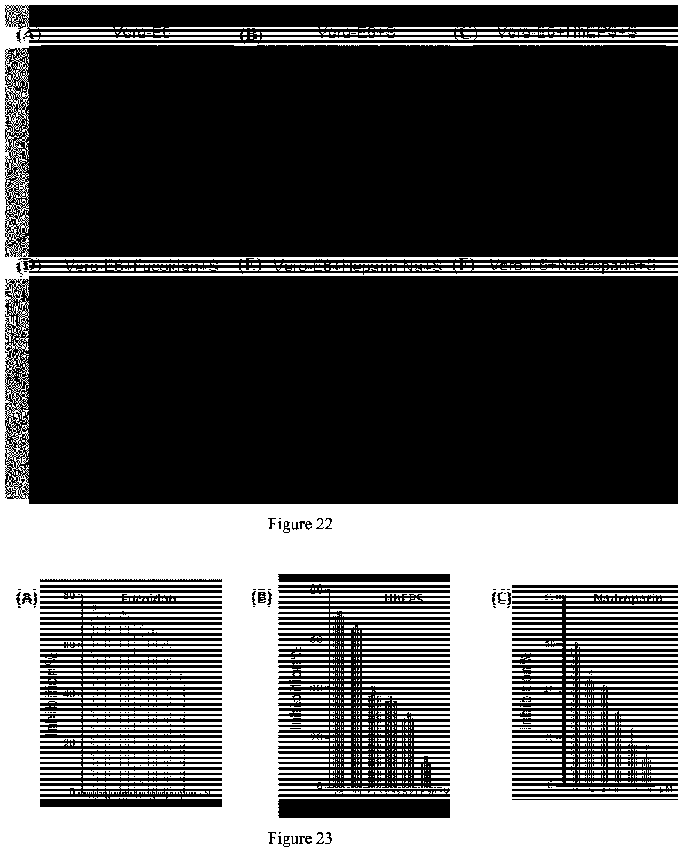

Figure 22 is a diagram of blocking the S1 subunit (the S1 protein) of SARS-CoV-2 to African green monkey kidney cells (Vero-E6 cells) by sugar chains of Example 6, and in the figure, A shows Vero-E6 cells; B shows the binding of the S1 protein to Vero-E6 cells; C shows an inhibitory effect of exopolysaccharide from *Haloarcula hispanica* (HhEPS) on the binding of the S1 protein to Vero-E6 cells; D shows an inhibitory effect of fucoidan (Fucoidan) on the binding of the S1 protein to Vero-E6 cells; E shows an inhibitory effect of sodium heparin sulfate (Heparin-Na) on the binding of the S1 protein to Vero-E6 cells; and F shows an inhibitory effect of nadroparin calcium (Nadroparin) on the binding of the S1 protein to Vero-E6 cells; and
Figure 23 is a diagram of inhibitory effects of sugar chains of Example 7 on pseudovirus invasion and infection of African green monkey kidney cells (Vero-E6 cells), and in the figure, A shows fucoidan (Fucoidan); B shows exopolysaccharide from *Haloarcula hispanica* (HhEPS); and C shows nadroparin calcium (Nadroparin).

### Detailed Description of the Invention

The present application will be further described below with reference to specific embodiments.

It should be noted that the terms "above", "below", "left", "right", "middle" and the like used herein are only for the convenience of description and understanding, and are not intended to limit the implementable scope. Changes or adjustments made to a relative relationship of technical features without substantially changing the technical content shall fall within the implementable scope of the present application.

Unless otherwise defined, all technical and scientific terms used herein have the same meaning as commonly understood by those skilled in the art to which the present application belongs. The term "and/or" used herein includes any and all combinations of one or more of associated items listed.

Experiments described without introducing the conditions are carried out in accordance with the conventional conditions or the conditions suggested by manufacturers. Reagents or instruments described without introducing manufacturers are commercially available common products.

The term "about" used herein is for providing flexibility in association with a given term, measurement or value. Those skilled in the art can readily determine the degree of flexibility of a specific variate.

The term "at least one of" used herein is intended to be synonymous with "one or more of'. For example, "at least one of A, B, C" expressly includes only A, only B, only C, and combinations of any two or more of A, B, and C.

Concentration, quantity, and other numerical data may be presented herein in range format. It should be understood that such range format is only for convenience and brevity, and should be flexibly construed to include not only values expressly stated as the limits of the range, but also all individual values or subranges subsumed within the range, as if each numerical value and subrange were expressly stated. For example, a numerical range of about 1 to about 4.5 should be construed to include not only the expressly stated limits of about 1 and about 4.5, but also individual numbers (e.g. 2, 3, and 4) and subranges (e.g. 1 to 3 and 2 to 4). The same principle is applicable to ranges with only one stated numerical value, such as "less than about 4.5", which should be construed to include all values and subranges that are less than about 4.5. In addition, this interpretation should applicable regardless of the range of the scope or features described.

Steps in any method or process claimed may be performed in any order and are not limited to the order defined in the claims.

The present application will be further described below with reference to specific examples.

### Example 1

### Experiment on enzymatic synthesis of functional oligosaccharides such as 6'-sialyllactose (6'-SL)

Related enzymes required by the synthesis of functional oligosaccharides were expressed and purified, and respectively subjected to activity analysis. Functional oligosaccharides were efficiently obtained by in vitro enzymatic synthesis, and then subjected to HMR and MS characterization.

The synthesis of 6'-sialyllactose (6'-SL) was taken as an example. A reaction system (10 mL) was composed of 3.6 mM sialic acid, 3 mM lactose, 3.6 mM CTP, Tris-HCl (pH=8.5, 100 mM), 20 mM MgCl₂, 2 µM NmCSS (CMP-sialic acid synthetase, Yu H, et al., Bioorganic & Medicinal Chemistry, 2004, 12, 6427-6435), 2 µM Pd26ST (α-2,6-sialyltransferase, Sun M, et al., Biotechnol Lett. 2008, 30, 671-676), and 2 µM PPA (pyrophosphatase, Li L, et al., Org. Lett., 2013, 15, 5528-5530). The conditions included that: the reaction system reacted at 140 rpm and 37°C for 48 h. After the reaction was completed, anhydrous ethanol with the same volume was added, and the mixture was concentrated, purified on Sepahadex G15, and further separated and purified by HPLC. Finally, a purified product, i.e. 6'-sialyllactose (6'-SL), was subjected NMR and mass spectrometry characterization.

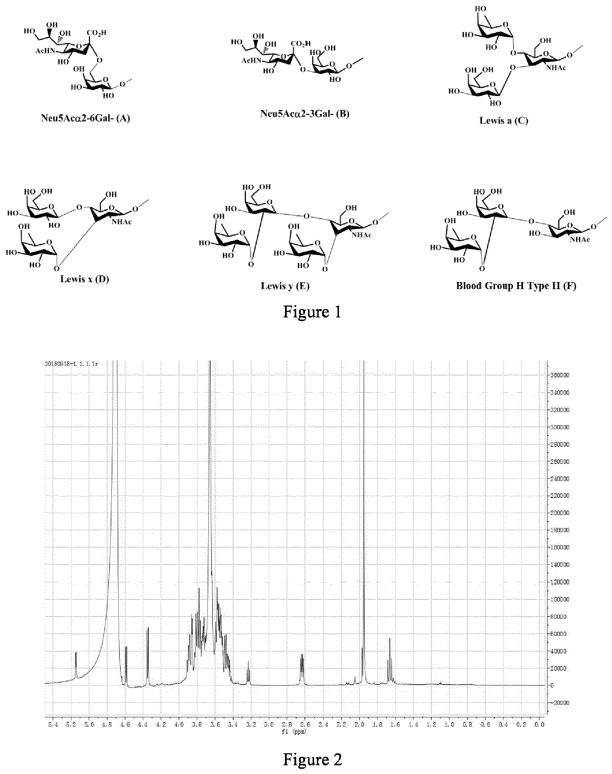

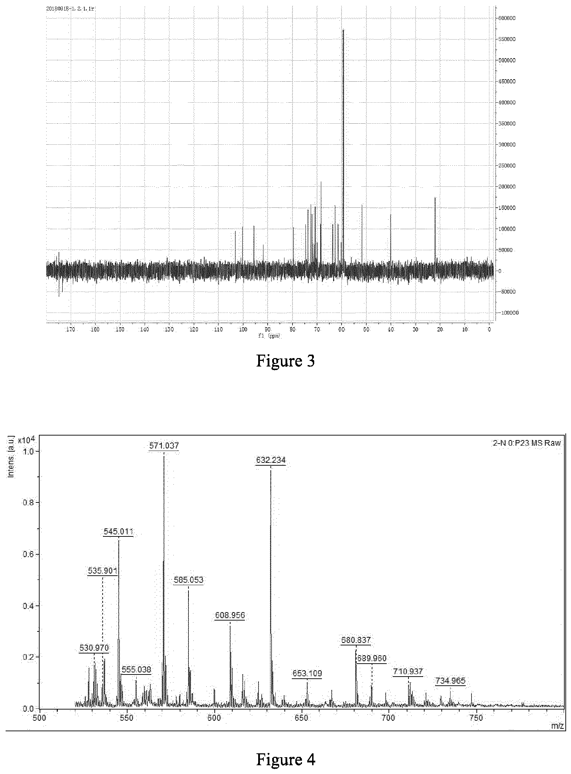

The purified product, i.e. 6'-SL, was subjected to ¹H NMR (see Figure 1), ¹³C NMR (see Figure 2), and mass spectrometry (see Figure 3) characterization. ¹HNMR (600 MHz, D₂O) δ 5.14 (d, *J*=6.0 Hz, H-1α), 4.59 (d, *J*=12.0 Hz, H-1β), 4.35 (d, *J*=12.0 Hz, 1H), 3.89-3.48 (m, 18H), 3.23 (t, *J*=6.0 Hz, 1H), 2.63 (dd, *J*=6.0, 12.0 Hz, 1H), 1.95 (s, 3H), 1.65 (t, *J*=12.0 Hz, 1H) (see Figure 1).

¹³C NMR (600 MHz, D₂O) δ 175.7, 174.9, 103.4, 100.4, 95.6, 91.8, 79.6, 74.6, 73.8, 72.5, 72.4, 71.8, 70.8, 68.5, 68.4 63.6, 62.7, 61.4, 60.3, 60.1, 51.8, 40.1, 22.1 (see Figure 2).

MALDI-TOF, the calculated molecular weight was 633.55 (the molecular formula of 6'-SL: C₂₃H₃₉NO₁₉), and the actual molecular weight was 632.234 (see Figure 3).

6'-sialyllactose (6'-SL) was a sugar chain containing the Neu5Acα2-6Gal building block at the non-reducing end.

### Example 2

### Cytotoxicity tests of a series of functional oligosaccharides

Effects of oligosaccharides (polysaccharides), such as sialic acid (Sia), 3'-sialyllactose (3'-SL), 6'-sialyllactose (6'-SL), 6'-*N*-acetyl sialyllactosamine (6'-SLN), chitosan oligosaccharide (COS), chondroitin sulfate (CS), sodium heparin sulfate (Heparin-Na), nadroparin calcium (Nadroparin), fucoidan (Fucoidan), and exopolysaccharide from *Haloarcula hispanica* (HhEPS), on 16HBE cells (bronchial epithelial cells), BEAS-2B cells (lung epithelial cells), and Vero-E6 (African green monkey kidney cells) were determined by MTT assays.

Specific experimental steps were as follows.
1) The above cells were respectively prepared into 20 mg/mL mother solutions.
2) Concentration gradients were set according to Table 1.

**Table 1 Concentration gradients**

| Concentration | 20 mg/mL mother solution (µL) | Volume of 1640 culture solution (µL) | Configured volume (µL) |
|---|---|---|---|
| Ctrl (Control) | 0 | 2000 | 2000 |
| 10 µg/mL | 1 | 1999 | 2000 |
| 50 µg/mL | 5 | 1995 | 2000 |
| 100 µg/mL | 10 | 1990 | 2000 |
| 200 µg/mL | 20 | 1980 | 2000 |
| 500 µg/mL | 50 | 1950 | 2000 |
| 1000 µg/mL | 100 | 1900 | 2000 |

2) The cell concentration was set to be 2×10⁵ cells/mL, and 150 µL of each prepared solution was inoculated into each well of a 96-well plate.
3) After the cells adhered to wells for 12 h, the culture solution was removed by suction, and 200 µL of oligosaccharide culture solutions at different concentrations were added to each well, and the same oligosaccharide culture solution was added to 6 wells.
4) 24 h after the oligosaccharide solutions at different concentrations were added, 20 µL of MTT solution at a concentration of 5 mg/mL was added to each well.
5) After the MTT solution was added, the cells were incubated for 3.5 h, and 150 µL of DMSO was added to each well.
6) After the cells were uniformly mixed with DMSO for 10 min, the 96-well plates were placed into a microplate reader and detected at a detection wavelength of 490 nm.
7) The relative cell viability was determined by comparing with values of the control at 490 nm.

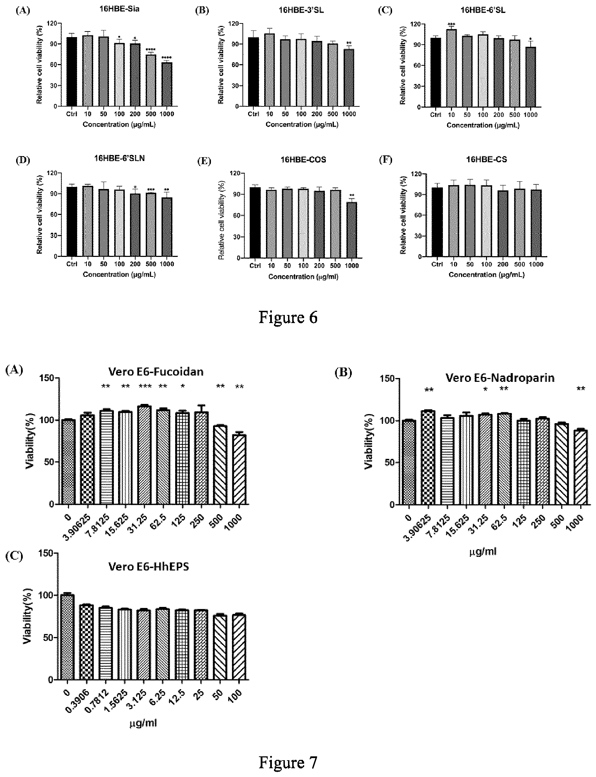

It can be known from results shown in Figure 5, Figure 6, and Figure 7 that most of the sugar chains have weak toxicity to lung epithelial cells (BEAS-2B cells), bronchial epithelial cells (16HBE cells), and African green monkey kidney cells (Vero-E6).

Among the sugar chains, 6'-*N*-acetyl sialyllactosamine (Neu5Acα2-6Galβ1-4GlcNAc, 6'-SLN) was an oligosaccharide containing the Neu5Acα2-6Gal building block.

### Example 3

Experiments on the binding of the S1 subunit of SARS-CoV-2 and DC-SIGN to an O-sugar chain chip were carried out in the present example. The O-sugar chain chip and HMO chip used in the present application were purchased from Nanjing Creative Biochip Co., Ltd.
1) Fluorescent labeling of S1 subunit and DC-SIGN sample solutions
1 mg/mL S1 subunit sample and 1 mg/mL DC-SIGN sample were respectively prepared. 100 µL of sample was added to 25 µL of labeling reaction solution at 5-fold diluted concentration (containing 130 mM NaHCO₃ and 50 mM NaCl, pH=8.2-8.3), an appropriate volume of Cy3-Se (cyanine 3 succinimidyl ester, an N-hydroxysuccinimide activated anthocyanidin 3 fluorescent dye) fluorescent dye (10 mg of Cy3-Se/1 mL of dimethyl sulfoxide), 0.5 µL of fluorescent dye was correspondingly added to the S1 subunit sample, 0.9 µL of fluorescent dye was correspondingly added to the DC-SIGN sample, and the mixtures were slightly shaken to form uniform reaction solutions.

**Table 2 Corresponding addition volume of fluorescent dye**

| Item | 1 (S1 subunit) | 2 (DC-SIGN) |
|---|---|---|
| Concentration (mg/mL) | 1 mg/mL | 1 mg/mL |
| CY3-Se (µL) | 0.5 | 0.9 |

2) The reaction solutions were shaken on a shaker for 1 h.
3) Dialysis after labeling: 125 µL of each reaction solution was injected into a mini dialysis tubing.
4) The reaction solutions were respectively stirred 3 times in a dialysis buffer solution (300 mL × 3) in the dark, and each stirring was performed for 1 h (after the last stirring, the reaction solutions were placed overnight).
5) The samples were taken out and respectively transferred into a new centrifuge tube.

### 3.2 Preparation of samples to be analyzed

Concentration of samples to be analyzed: the 2 samples were denoted as No. 1 and No. 2 samples, respectively. The No. 1 sample referred to the S1 subunit, and the No. 2 sample referred to DC-SIGN. The above No. 1 and No. 2 samples were subjected to serial dilution to form samples at different test concentrations.

Specifical dilutions were as follows: at the first dilution, the samples were diluted to a concentration of 10 µg/mL (the volume of each sample was 250 µL); at the second dilution, the samples were subjected to 1-fold dilution to a concentration of 5 µg/mL (the volume of each sample was 250 µL); at the third dilution, the samples were subjected to 1-fold dilution to a concentration of 2.5 µg/mL (the volume of each sample was 250 µL); and at the forth dilution, the samples were subjected to 1-fold dilution to a concentration of 1.25 µg/mL (the volume of each sample was 250 µL).

The corresponding arrangement of the samples at different test concentrations on an 8-subarray microarray chip is shown in Table 3.

**Table 3 Arrangement of samples on an 8-subarray microarray chip**

| Serial No. of samples | Sugar chain concentration | Volume |
|---|---|---|
| No. 1 sample (S1 subunit) | 10 µg/mL | 250 µL |
| No. 1 sample (S1 subunit) | 5 µg/mL | 250 µL |
| No. 1 sample (S1 subunit) | 2.5 µg/mL | 250 µL |
| No. 1 sample (S1 subunit) | 1.25 µg/mL | 250 µL |
| No. 2 sample (DC-SIGN) | 10 µg/mL | 250 µL |
| No. 2 sample (DC-SIGN) | 5 µg/mL | 250 µL |
| No. 2 sample (DC-SIGN) | 2.5 µg/mL | 250 µL |
| No. 2 sample (DC-SIGN) | 1.25 µg/mL | 250 µL |

### 3.3. Chip analysis

### Part I: blocking

The microarray chip was treated in a clean and dry environment. Gloves were required when touching the surface of the chip.

Before being opened, the chip package was placed at room temperature for about 20 min. Then, the package was opened, and a chip to be used was taken out, encapsulated into a reusable pouch with a desiccant inside, and stored in a refrigerator at -20°C.

Fences for analysis were mounted on the chip. A blocking buffer solution was added to each subarray. For an 8-subarray chip (referring to a glass substrate with 8 printed sugar chain areas, which contain the same sugar chain and are used to detect 8 different samples at the same time), 200 µL of buffer solution added to each subarray.

The subarrays were sealed with a plastic film to prevent evaporation of the blocking buffer solution, and incubated on a rotary shaker (at 60 rpm) for 30 min.

### Part II: binding experiment

The samples were centrifuged on a centrifuge to avoid increasing particles on the chip.

A pipette was carefully put at the corner of each subarray to remove the blocking buffer solution from each subarray without touching the surface of the microarray chip.

The samples to be analyzed were added to each subarray immediately. For the 8-subarray chip, 200 µL of sample was added to each subarray. The sample solution should cover the entire area of each subarray without producing air bubbles in the sample solution.

The microarray chip was sealed with a plastic film to prevent evaporation of the sample solution, and covered with aluminum foil and incubated in the dark for 1 h (on a shaker at 80 rpm).

5) During the experiment, especially during the long incubation, the surface of the chip was prevented from being dried. A sealing effect of the plastic film should be ensured.

### Part III: final washing and drying

A pipette was carefully put at the corner of each subarray to take the sample from each subarray without touching the surface of the microarray chip.

A washing buffer solution was added to each subarray. For the 8-subarray chip, 200 µL of buffer solution was added to each subarray. The microarray chip was sealed with a plastic film, and incubated on a shaker (at 80 rpm) for 5 min. The washing buffer solution was removed immediately by using a pipette, and the above steps were repeated.

After the washing buffer solution was completely removed, the fences for analysis were unmounted from the chip, the glass slide was immersed in a washing buffer solution, and the chip was incubated on a shaker (at 80 rpm) for 10 min.

The glass slide was immersed in deionized water, and the chip was incubated on a shaker (at 80 rpm) for 2 min.

Before data scanning, the chip was dried in a clean and dust-free environment.

### Part IV: data reading and analysis

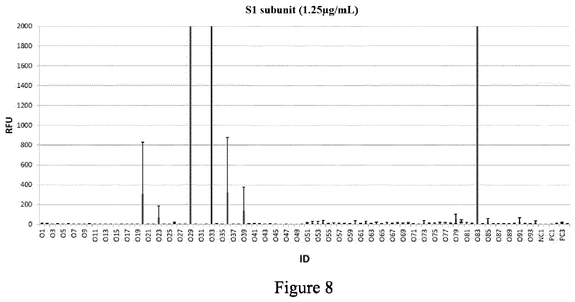

Figure 8 is a diagram of the binding of the S1 subunit of SARS-CoV-2 to the O-sugar chain chip. It can be seen from the results shown in Figure 8 that when the concentration of the S1 subunit is 1.25 µg/mL, some O-sugar chains bind to the S1 subunit of SARS-CoV-2 to a certain extent.

The sugar chains include O20 (Fucα1-2Galβ1-4(Fucα1-3)GlcNAcβ1-3Galβ1-3GalNAcα), O33 (Fucα1-2Galβ1-4(Fucα1-3)GlcNAcβ1-3GalNAcα), O23 (GlcNAcβ1-6(Galβ1-3)GalNAcα), O29 (Neu5Acα2-3Galβ1-4GlcNAcβ1-3GalNAcα), O36 (Galα1-3(Fucα1-2)Galβ1-4GlcNAcβ1-3NAcα), O39 (GlcNAcβ1-6(GlcNAcβ1-3)GalNAcα), and O83 (Neu5Acα2-6Galβ1-4GlcNAcβ1-3(Neu5Acα2-6Galβ1-3GlcNAcβ1-6)GalNAcα).

**Table 4 Binding of the S1 subunit of a virus to an O-sugar chain chip**

| Sugar chain | RFU |
|---|---|
| O20 | 304.5 |
| O23 | 69.84 |
| O29 | 2866.34 |
| O33 | 6320.17 |
| O36 | 320.5 |
| O39 | 136.5 |
| O83 | 6012.67 |

Among the sugar chains, O20 and O33 are sugar chains containing the Lewis y building block at the non-reducing end, O83 is a sugar chain containing the Neu5Acα2-6Gal building block at the non-reducing end, O29 is a sugar chain containing the Neu5Acα2-3Gal building block at the non-reducing end, and O23 and O39 are sugar chains containing the GlcNAcβ1-6 building block at the non-reducing end. Therefore, it can be determined from the above results that HMOs, such as 3'-sialyllactose containing Neu5Acα2-6Gal at the non-reducing end and 3'-sialyllactosamine containing Neu5Acα2-3Gal at the non-reducing end, can also bind to the S1 subunit.

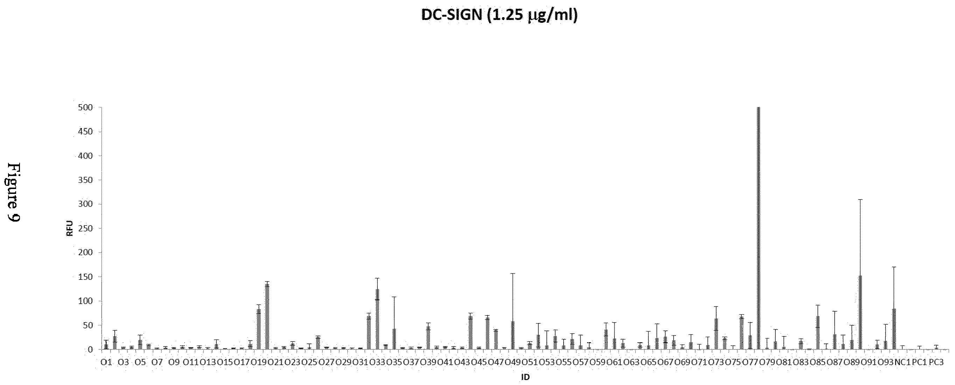

Figure 9 is a diagram of the binding of the DC-SIGN protein to the O-sugar chain chip. It can be seen from the results shown in Figure 9 that some O-sugar chains bind to the DC-SIGN protein of a receptor to a certain extent.

**Table 5 Binding of DC-SIGN to an O-sugar chain chip**

| Sugar chain | RFU |
|---|---|
| O19 | 83.33 |
| O20 | 135 |
| O32 | 68.83 |
| O33 | 124.5 |
| O44 | 69 |
| O46 | 65.83 |

The sugar chain include 019 (Galβ1-4(Fucα1-3)GlcNAcβ1-3Galβ1-3GalNAcα), O20 (Fucα1-2Galβ1-4(Fucα1-3)GlcNAcβ1-3Galβ1-3GalNAcα), O32 (Galβ1-4(Fucα1-3)GlcNAcβ1-3GalNAcα), O33 (Fucα1-2Galβ1-4(Fucα1-3)GlcNAcβ1-3GalNAcα), O44 (Galβ1-4(Fucα1-3)GlcNAcβ1-3GalNAcα), and O46 (Fucα1-2Galβ1-4(Fucα1-3)GlcNAcβ1-6GalNAcα.

Among the sugar chains, 019, O32, and O44 are sugar chains containing the Lewis x building block at the non-reducing end, and O20, O33, and O46 are sugar chains containing the Lewis y building block at the non-reducing end.

It can be known from the above results that O20 (Fucα1-2Galβ1-4(Fucα1-3)GlcNAcβ1-3Galβ1-3GalNAcα) and O33 (Fucα1-2Galβ1-4(Fucα1-3)GlcNAcβ1-3GalNAcα) are sugar chains that can bind to not only the S1 subunit, but also DC-SIGN.

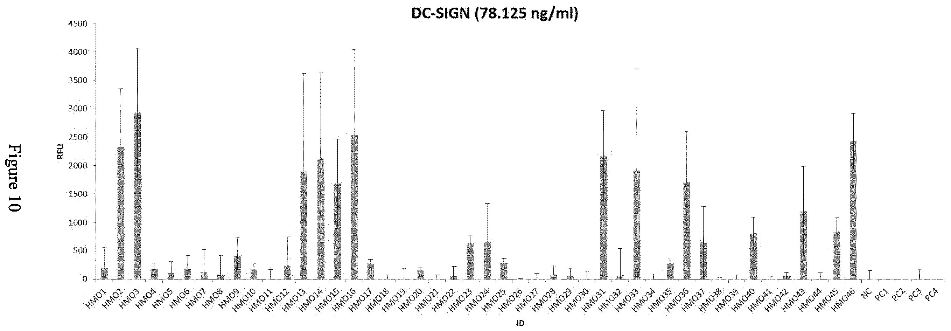

Figure 10 is a diagram of the binding of DC-SIGN of a receptor to an HMO chip. It can be seen from the results shown in Figure 10 that some HMOs bind to DC-SIGN to a certain extent.

The HMOs contain the Lewis a, Lewis x, Lewis y or Blood Group H type II building block at the non-reducing end, such as HMO13 (Galβ1-3(Fucα1-4)GlcNAcβ1-3Galβ1-4Glc), HMO14 (Galβ1-4(Fucα1-3)GlcNAcβ1-3Galβ1-4Glc), HMO15 (Fucα1-2Galβ1-4GlcNAcβ1-3Galβ1 -4Glc), HMO16 (Fucα1-2Galβ1-4(Fucα1-3)GlcNAcβ1-3Galβ1-4Glc), HMO31 (GlcNAcβ1-6[Galβ1-4(Fucα1-3)GlcNAcβ1-3]Galβ1-4Glc), HMO33 (GlcNAcβ1-6[Fucα1-2Galβ1-4(Fucα1-3)GlcNAcβ1-3]Galβ1-4Glc), HMO36 (Galβ1-4(Fucα1-3)GlcNAcβ1-6(GlcNAcβ1-3)Galβ1-4Glc), HMO40 (Galβ1-4GlcNAcβ1-6(Fucα1-2Galβ1-4GlcNAcβ1-3)Galβ1-4Glc), HMO43 (Galβ1-4(Fucα1-3)GlcNAcβ1-6(Galβ1-4GlcNAcβ1-3)Galβ1-4Glc), HMO45 (Fucα1-2Galβ1-4GlcNAcβ1-6(Fucα1-2Galβ1-4GlcNAcβ1-3)Galβ1-4Glc), HMO46 (Fucα1-2Galβ1-4(Fucα1-3)GlcNAcβ1-6(Fucα1-2Galβ1-4(Fucα1-3)GlcNAcβ1-3)Galβ1 -4Glc), HMO2 (Galβ1-4(Fucα1-3)Glc), and HMO3 (Fucα1-2Galβ1-4Glc).

**Table 6 Binding of DC-SIGN to an HMO chip**

| Sugar chain | RFU |
|---|---|
| HMO2 | 2329.09 |
| HMO3 | 2930.75 |
| HMO13 | 1896.5 |
| HMO14 | 2125.34 |
| HMO15 | 1683.84 |
| HMO16 | 2537.92 |
| HMO31 | 2175.59 |
| HMO33 | 1913.59 |
| HMO36 | 1707.42 |
| HMO40 | 801.5 |
| HMO43 | 1196.59 |
| HMO45 | 838.42 |
| HMO46 | 2425.92 |

Figure 1 is a diagram of structural formulas of Neu5Acα2-6Gal, Neu5Acα2-3Gal, Lewis a, Lewis x, Lewis y, and Blood Group H type II, and in the figure, 1A shows Neu5Acα2-6Gal, 1B shows Neu5Acα2-3Gal, 1C shows Lewis a, 1D shows Lewis x, 1E shows Lewis y, and 1F shows Blood Group H type II.

### Example 4

Experiments on the binding of the S1 protein (or the S1 subunit) of SARS-CoV-2 to various oligosaccharides were carried out in the present example. An MO NT.115 microscale thermophoresis measurement instrument purchased from Nano Temper was used for detection, and experimental steps were as follows.
1) First, the S1 protein was subjected to fluorescent labeling. In the present experiment, a Monolith^{™} RED-NHS 2^{nd} generation protein labeling kit was used for labeling.
2) A buffer solution for the S1 protein was replaced. 100 µg of S1 protein (1 mg/mL) expressed in mammalian cells was taken, the column A of the kit was used to replace the protein dissolution buffer solution with a labeling buffer solution (containing 130 mM NaHCO₃ and 50 mM NaCl, pH=8.2-8.3).
3) 25 µL of dimethyl sulfoxide (DMSO) was used to dissolve a RED-NHS dye of the kit.
4) 5 µL of RED-NHS dye was diluted and uniformly mixed with 5 µL of labeling buffer solution.
5) 90 µL of S1 protein was added and uniformly mixed with the fluorescent dye, and labelled in the dark at 37°C for 30 min.
6) The labelled protein was purified by using the column B, and 450 µL of purified protein was obtained.
7) Oligosaccharides used in the experiment were subjected to serial dilutions with ddH₂O, each oligosaccharide was diluted at 16 concentration gradients, and the initial concentration of each oligosaccharide was as follows:
   sodium heparin sulfate (Heparin-Na): 83.33 µM;
   sodium chondroitin sulfate (Chondroitin sulfate-Na): 40 mg/mL, 285.75 µM;
   exopolysaccharide from *Haloarcula hispanica* (HhEPS, EPS from *Haloarculahispanica*): 22 mg/mL, 10 µM;
   fucoidan (Fucoidan): 30 mg/mL, 60 µM;
   3-fucosyllactose (Galβ1-4(Fucα1-2)Glc, 3-FL): 1 mg/mL, 2049 µM;
   2'-fucosyllactose (Fucα1-2Galβ1-4Glc, 2'-FL): 5 mg/mL, 10.24 mM; and
   nadroparin calcium (Nadroparin): 444.33 µM.

The labelled protein was mixed with the oligosaccharide solution with the same volume. Generally, 10 µL of labelled protein was mixed with 10 µL of oligosaccharide solution, and 20 µL of reaction system was obtained.

The oligosaccharide-protein mixed solutions at 16 concentration gradients were sampled by using capillaries. 7 µL of sample was taken each time, and 20 µL of reaction solution was used in three repeated experiments.

The 16 capillaries with samples were placed on the lanes of the MO NT.115 microscale thermophoresis measurement instrument in order to be subjected to a protein-oligosaccharide binding experiment.

Dissociation constants (K_{d}) of the binding of the S1 protein to the sugar chains were calculated from obtained data. The dissociation constants (K_{d}) of the binding of the S1 protein to the sugar chains are shown in Table 7.

**Table 7 Dissociation constants (K_{d}) of the binding of the S1 protein to various sugar chains**

| Sugar chain | Kd (µM) |
|---|---|
| Sodium heparin sulfate (Heparin-Na) | 1.25 |
| Sodium chondroitin sulfate (Chondroitin sulfate-Na) | 19 |
| Exopolysaccharide from *Haloarcula hispanica* (HhEPS, EPS from *Haloarculahispanica*) | 3.77 |
| Fucoidan (Fucoidan) | 1.61 |
| 3-fucosyllactose (Galβ1-4(Fucα1-2)Glc, 3-FL) | 7.1 |
| 2'-fucosyllactose (Fucα1-2Galβ1-4Glc, 2'-FL) | 71 |
| Nadroparin calcium (Nadroparin) | 6.2 |

It can be known from the data in Table 7 that the affinity of sugar chains, such as sodium heparin sulfate, fucoidan, exopolysaccharide of *Haloarcula hispanica*, and 3-fucosyllactose, with the S1 protein is relatively strong, and the affinity of sodium chondroitin sulfate and 2'-fucosyllactose with the S1 protein is relatively weak.

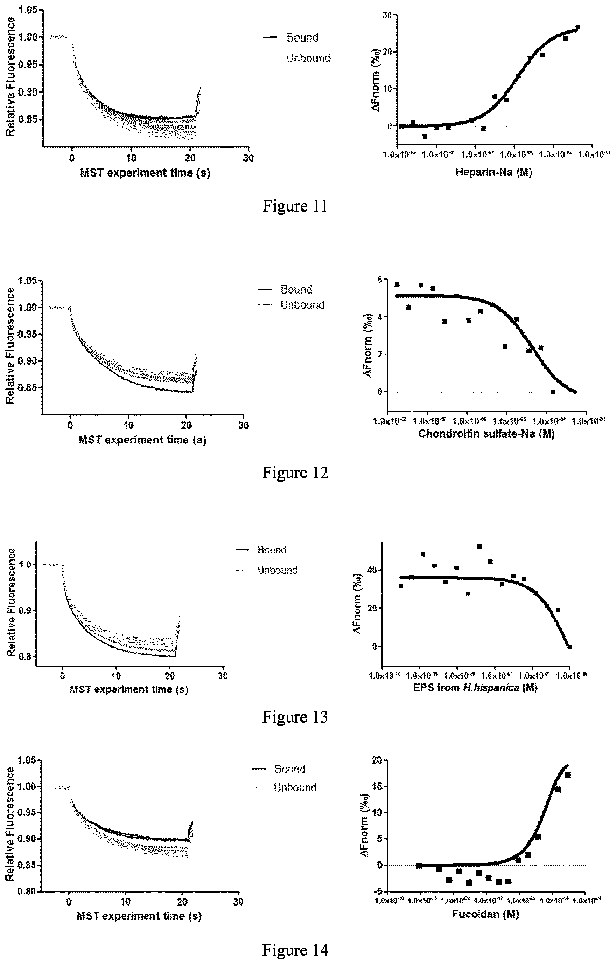

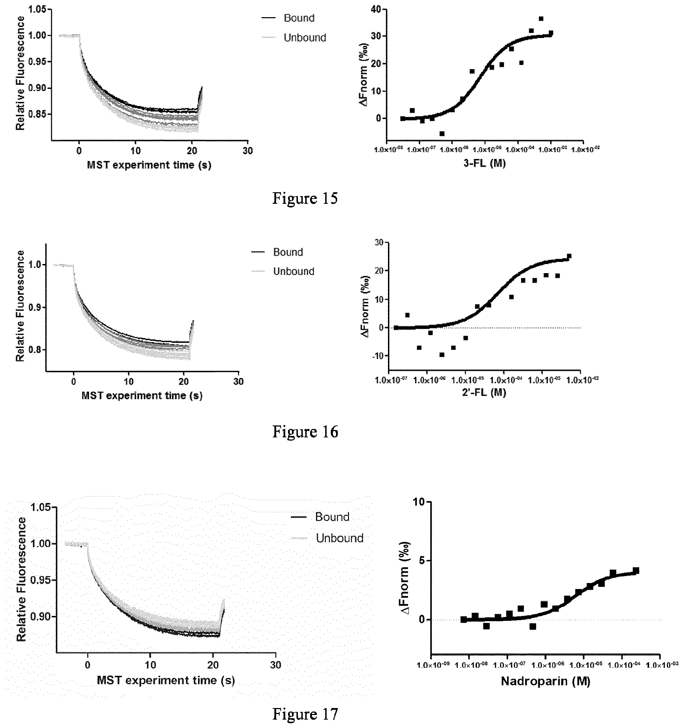

Figure 11 is a diagram of the binding of the S1 subunit of the virus to heparin sulfate in the experiment; Figure 12 is a diagram of the binding of the S1 subunit of the virus to chondroitin sulfate; Figure 13 is a diagram of the binding of the S1 subunit of the virus to exopolysaccharide from *Haloarcula hispanica*; Figure 14 is a diagram of the binding of the S1 subunit to fucoidan; Figure 15 is a diagram of the binding of the S1 subunit to 3-fucosyllactose; Figure 16 is a diagram of the binding of the S1 subunit to 2'-fucosyllactose; and Figure 17 is a diagram of the binding of the S1 subunit to nadroparin calcium.

In Figure 11 to Figure 17, the left diagrams show thermophoresis curves of the binding of the sugar chains at different concentrations to the S1 subunit, and the right diagrams show curves of the binding of the sugar chains at different concentrations to the S1 subunits. In the left diagrams, the x-axis refers to time, and y-axis refers to relative fluorescence intensities; and in the right diagrams, the x-axis refers to concentrations of the sugar chains, and the y-axis refers to one thousandth of differences between fluorescence intensities of the binding of the sugar chains at different concentrations to the S1 subunit and a fluorescence intensity of the binding of the sugar chains at the lowest concentration to the S1 subunit.

It can be known from Figure 11 to Figure 17 that the 6 sugar chains in the above example can all specifically bind to the S1 subunit of the virus in the experiment, but exhibits different concentration dependencies upon binding.

### Example 5

Experiments on blocking of the binding of the S1 subunit (the S1 protein) of SARS-CoV-2 to human bronchial epithelial cells by sugar chains were carried out in the present example. Experimental steps were as follows.
1) 16HBE cells (bronchial epithelial cells) were inoculated into a 24-well cell culture plate with a glass bottom (Cellvis), 1 mL of culture medium (containing 90% 1640 culture medium, 10% fetal calf serum, and 1% double antibody (Pen Strep: 100 U/mL penicillin and 10 µg/mL streptomycin)) was added to each well, after being cultured for 24-48 h, the cells covered the bottom of each well, and next step was performed.
2) The cell culture solution in the 24-well plate was removed by suction, and each well was washed 3 times with PBS (pH=7.4, 1 mL × 3).
3) 200 µL of each sugar chain solution, i.e. 40 µM chondroitin sulfate and 20 µM fucoidan, was added to each well to incubate the cells, 200 µL of PBS was added to a control (without any saccharide), and the cells were incubated for 1 h.
4) After 1 h, 100 µL of fluorescently labeled (using a Monolith^{™} RED-NHS 2^{nd} generation protein labeling kit that was purchased from Nano Temper) S1 protein (10 µg/mL) was added to each well, and the cells were incubated for another 1 h.
5) Solution in each well was removed by suction, and each well was washed 3 times with PBS (500 µL × 3).
6) 200 µL of paraformaldehyde (4%) tissue fixation solution was added to each well to fix the cells for 30 min.
7) Solution in each well was removed by suction, and each well was washed 3 times with PBS (500 µL × 3).
8) 1 µg/mL of DAPI (4',6-diamidino-2-phenylindole) dye was added to each well to stain cell nucleus for 10 min.
9) Solution in each well was removed by suction, and each well was washed 3 times with PBS (500 µL × 3).
10) 300 µL of PBS was added to each well, and the binding of the S1 protein (red light) to the cells (blue light) was observed under a laser scanning confocal microscope.

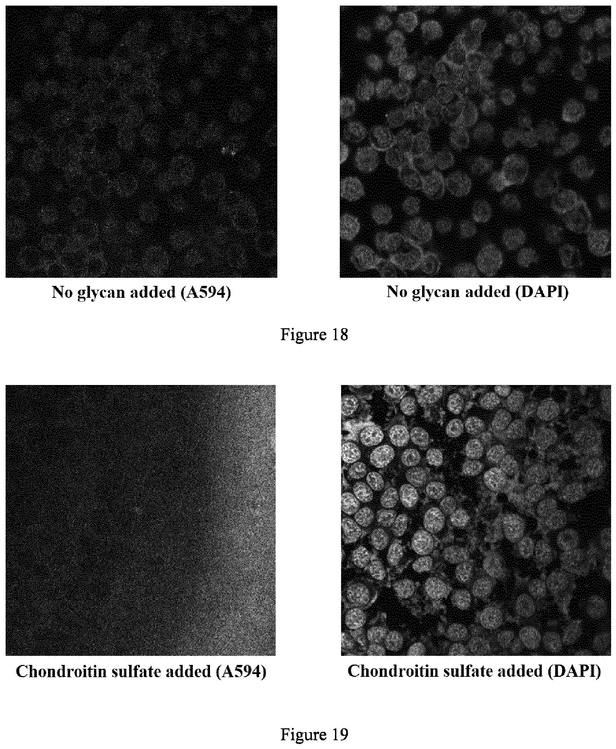

Figure 18 is a diagram of the control without any sugar chain; Figure 19 is a diagram of blocking the binding of the S1 subunit to 16HBE cells (bronchial epithelial cells) by chondroitin sulfate; and Figure 20 is a diagram of blocking the binding of the S1 subunit to 16HBE cells (bronchial epithelial cells) by fucoidan.

In Figure 18 to Figure 20, the left diagrams show observation results of RED staining, and the right diagrams show observations results of DAPI staining. As shown in Figure 18 to Figure 20, the S1 protein directly binds to the cells, and RED and DAPI staining conditions are respectively observed by using red light and blue light. The results show that clear cell morphologies can be observed at both wavelengths, and the two cell morphologies coincide, which indicates that the S1 protein successfully binds to 16HBE cells (bronchial epithelial cells). On the contrary, after chondroitin sulfate or fucoidan is added, the clear cell morphology can be observed from the DAPI staining condition, and the cellular outline cannot be seen under red light, which indicates that the S1 protein does not bind to the cells, and also proves that chondroitin sulfate and fucoidan successfully block the binding of the S1 protein to bronchial epithelial cells.

### Example 6

Experiments on blocking the binding of the S1 subunit (the S1 protein) of SARS-CoV-2 to lung epithelial cells (BEAS-2B cells) and African green monkey kidney cells (Vero-E6 cells) by sugar chains were carried out in the present example. Experimental steps were as follows.
(1) The cells were inoculated into a 24-well cell culture plate with a glass bottom (1×10⁵ cells/well), 1 mL of culture medium was added to each well, after being cultured for 24-48 h, the cells covered the bottom of each well, and next step was performed.
(2) The cell culture solution in the 24-well plate was removed by suction, and each well was washed 3 times with PBS (1 mL × 3).
(3) 300 µL of 4% paraformaldehyde (PFA) solution was added to each well to fix the cells at room temperature for 1 h.
(4) The fixation solution was removed by suction, and each well was washed 3 times with PBS (1 mL × 3).
(5) Sugar chains were added to the cells to pre-protect the cells, and the cells were incubated at 37°C for 1 h.
(6) After 1 h, the S1 subunit (1 µM) was added to each well, and the cells were incubated at 37°C for 1 h.
(7) The solution in each well was removed by suction, and each well was washed 3 times with PBS (1 mL × 3).
(8) 200 µL of Mouse anti-SARS-CoV-2 mAb (Sino Biological, 40592-MM57) diluted with an HBSSA solution in a ratio of 1: 1000 was added to each well, and the cells were incubated at 37°C for 1 h.
(9) The primary antibody solution was removed by suction, and each well was washed 3 times with PBS (1 mL × 3).
(10) 200 µL of Goat anti-Mouse IgG (H+L) Cross-Adsorbed Secondary Ab (Alexa Fluor 488) diluted with an HBSSA solution in a ratio of 1: 1000 was added to each well, and the cells were incubated at 37°C for 1 h.
(11) The solution in each well was removed by suction, and each well was washed 3 times with PBS (500 µL × 3).
(12) 1 µg/mL of DAPI dye was added to each well to stain the cells for 10 min.
(13) The solution in each well was removed by suction, and each well was washed 3 times with PBS (500 µL × 3).
(14) The cells were observed under a laser scanning confocal microscope at 405 nm (DAPI) and 490 nm (FITC), respectively, and inhibitory effects of the sugar chains on the binding of the S1 protein to the cells were evaluated.

Figure 21 is a diagram of blocking the binding of the S1 subunit to BEAS-2B cells (lung epithelial cells) by the sugar chains; and Figure 22 is a diagram of blocking the binding of the S1 subunit to Vero-E6 cells (African green monkey kidney cells) by the sugar chains.

It can be seen from Figure 20 and Figure 21 that sugar chains, such as exopolysaccharide from *Haloarcula hispanica* (HhEPS), fucoidan (Fucoidan), sodium heparin sulfate (Heparin-Na), and nadroparin (Nadroparin), can block the binding of the S1 subunit to BEAS-2B cells and Vero-E6 cells.

### Example 7

### Experiments on inhibitory effects of oligosaccharides on pseudovirus invasion and infection of Vero-E6 cells

(1) Vero-E6 cells were inoculated into a 48-well plate (5×10⁴ cells/well), 1 mL of culture medium was added to each well, after being cultured for 24 h, the cells covered the bottom of each well, and the next step was performed.
(2) Viroid of SARS-CoV-2 was amplified in 293T cells and subjected to TCID50 titration.
(3) The culture medium in the 48-well plate was removed by suction, and the 48-well plate was washed twice with DMEM (without serum).
(4) Sugar chains were subjected to gradient dilution with fresh DMEM (containing 10% FBS), and 200 µL of diluted sugar chain was added to each well of the 48-well plate, and the cells were incubated at 37°C for 2 h.
(5) A pseudovirus solution was added to the 48-well plate, and the cells were incubated at 37°C for 24 h.
(6) A supernate was taken, and each well was washed twice with PBS (500 µL × 2).
(7) 100 µL of pancrelipase solution was added to each well, and the cells were digested for 3-5 min.
(8) 100 µL of DMEM containing 2% FBS was added to each well, and the cells were suspended by blowing and beating and then transferred into a flow tube.
(9) Proliferation of the pseudovirus was analyzed by using a flow cytometer, and inhibitory effects of the sugar chains on the pseudovirus were evaluated.

Figure 23 is a diagram of inhibitory effects of the sugar chains on pseudovirus invasion and infection of Vero-E6 cells.

It can be seen from Figure 23 that sugar chains, such as fucoidan (Fucoidan), exopolysaccharide from *Haloarcula hispanica* (HhEPS), and nadroparin (Nadroparin), exhibit an inhibition activity to pseudovirus invasion and infection of the cells, and the highest inhibition rate is)[y1].

### Example 8

A mouthwash for preventing and/or treating 2019-nCoV infection was prepared, in which the concentration of an active ingredient was 0.01%-1% (W/V), and the active ingredient contained the following sugar chain components:
Fucα1-2Galβ1-4(Fucα1-3)GlcNAcβ1-3Galβ1-3GalNAcα,
Fucα1-2Galβ1-4(Fucα1-3)GlcNAcβ1-3GalNAcα, sulfated fucoidan, HMOs, ginsenosides, chitosan oligosaccharide, heparin sulfate, heparan sulfate oligosaccharide,
chondroitin sulfate, chondroitin sulfate oligosaccharide, dermatan sulfate, keratan sulfate, hyaluronic acid, hyaluronic acid oligosaccharide, fucoidan, fucose oligosaccharide, protein N/O-sugar chains, and exopolysaccharide from *Haloarcula hispanica.*

Other ingredients of the mouthwash were water or ingredients applicable to mouthwash.

### Example 9

A mouthwash for preventing and/or treating 2019-nCoV infection was prepared, in which the concentration of an active ingredient was 0.01%-1% (W/V), and the active ingredient contained the following sugar chain components:
Fucα1-2Galβ1-4GlcNAcβ1-3Galβ1-4Glc,
Fuca1-2Galβ1-4GlcNAcβ1-6(Fucα1-2Galβ1-4GlcNAcβ1-3)Galβ1-4Glc, sodium heparin sulfate, fucoidan, exopolysaccharide from *Haloarcula hispanica*, and 3-fucosyllactose.

Other ingredients of the mouthwash were glycerol or ingredients applicable to mouthwash.

### Example 10

A nasal wash for preventing and/or treating coronavirus infection was prepared, in which the concentration of an active ingredient was 0.01%-1% (W/V), and the active ingredient contained the following sugar chain components:
Neu5Acα2-6Galβ1-4GlcNAcβ1-3(Neu5Acα2-6Galβ1-3GlcNAcβ1-6)GalNAc,
6'-sialyllactose (Neu5Acα2-6Galβ1-4Glc), 6'-*N*-acetyl sialyllactosamine (Neu5Acα2-6Galβ1-4GlcNAc),
Fucα1-2Galβ1-4(Fucα1-3)Galβ1-4GlcNAcβ1-3Galβ1-3GalNAcα,
Fucα1-2Galβ1-4(Fucα1-3)GlcNAcβ1-3Galβ1-3GalNAcα,
Fucα1-2Galβ1-4(Fucα1-3)GlcNAcβ1-6GalNAcα,
Fucα1-2Galβ1-4(Fucα1-3)GlcNAcβ1-3Galβ1-4Glc, and Fucα1-2Galβ1-4(Fucα1-3)GlcNAcβ1-6(Fucα1-2Galβ1-4(Fucα1-3)GlcNAcβ1-3)Galβ1-4Glc.

Other ingredients of the nasal wash were glycerol or ingredients applicable to nasal wash.

### Example 11

A pharmaceutical composition for preventing and/or treating 2019-nCoV infection, in which the concentration of an active ingredient was 0.01%-1% (W/V), and the active ingredient contained the following sugar chain components:
Fucα1-2Galβ1-4(Fucα1-3)Galβ1-4GlcNAcβ1-3Galβ1-3GalNAcα,
Fucα1-2Galβ1-4(Fucα1-3)GlcNAcβ1-3Galβ1-3GalNAcα,
Fucα1-2Galβ1-4(Fucα1-3)GlcNAcβ1-6GalNAcα,
Fucα1-2Galβ1-4(Fucα1-3)GlcNAcβ1-3Galβ1-4Glc,
Fucα1-2Galβ1-4(Fucα1-3)GlcNAcβ1-6(Fucα1-2Galβ1-4(Fucα1-3)GlcNAcβ1-3)Galβ1-4Glc, Fucα1-2Galβ1-4GlcNAcβ1-3Galβ1-4Glc, and Fucα1-2Galβ1-4GlcNAcβ1-6(Fucα1-2Galβ1-4GlcNAcβ1-3)Galβ1-4Glc.

Other ingredients of the pharmaceutical composition are preferably water, and the pharmaceutical composition was delivered to the lung with the assistance of a gasifying agent.

The above content is a schematic description of the present application and its implementation modes, and the description is not restrictive. Implementation modes shown in the embodiments are only some of the implementation modes of the present application, and the actual implementation modes are not limited thereto. Therefore, any structural modes and embodiments similar to the technical solutions designed by those of ordinary skill in the art inspired by the implementation modes without creativity and without departing from the purpose of the present invention shall fall within the scope of protection of the present application.

## Claims

1. A sugar chain, **characterized in that** the sugar chain contains any one or more of Neu5Acα2-N₁Gal building blocks, and/or any one or more of xFuc-N₁Gal-N₁(xFuc-N₁)GlcNAc building blocks, at the non-reducing end, where, x=0 or 1, and N₁=1, 2, 3, 4 or 6.

2. The sugar chain according to claim 1, **characterized in that** in the xFuc-N₁Gal-N₁(xFuc-N₁)GlcNAc building blocks, a glycosidic bond formed between any two adjacent monosaccharides is an α1 or β1 glycosidic bond.

3. The sugar chain according to claim 1 or 2, **characterized in that** in the xFuc-N₁Gal-N₁(xFuc-N₁)GlcNAc building blocks, a glycosidic bond formed between Fuc and Gal is an α1 or β1 glycosidic bond, a glycosidic bond formed between Gal and GlcNAc is an α1 or β1 glycosidic bond, and a glycosidic bond formed between Fuc and GlcNAc is an α1 or β1 glycosidic bond.

4. The sugar chain according to any one of claims 1 to 3, **characterized in that** the xFuc-N₁Gal-N₁(xFuc-N₁)GlcNAc building blocks comprise xFuc-N₁Gal-(xFuc-N₁)GlcNAcα1 or xFuc-N₁Gal-(xFuc-N₁)GlcNAcβ1, where, x=0 or 1, and N₁=1, 2, 3, 4 or 6.

5. The sugar chain according to any one of claims 1 to 4, **characterized in that** the xFuc-N₁Gal-N₁(xFuc-N₁)GlcNAc building blocks comprise xFucα1-N₁Galβ1-(xFucα1-N₁)GlcNAcβ1, where, x=0 or 1, and N₁=1, 2, 3, 4 or 6.

6. The sugar chain according to any one of claims 1 to 5, **characterized in that** the Neu5Ac-Gal building blocks comprise the Neu5Acα2-6Gal or Neu5Acα2-3Gal building block, and the xFuc-N₁Gal-N₁(xFuc-N₁)GlcNAc building blocks comprise the Lewis a, Lewis x, Lewis y or Blood Group H building block.

7. The sugar chain according to any one of claims 1 to 6, **characterized in that** the sugar chain contains A and B building blocks at the non-reducing end, the A building block is any one or more of Lewis a, Lewis x, Lewis y, and Blood Group H, and the B building block is the Neu5Acα2-6Gal and/or Neu5Acα2-3Gal building blocks.

8. The sugar chain according to any one of claims 1 to 7, **characterized in that** the sugar chain contains a combination of at least two of the Lewis a, Lewis x, Lewis y, and Blood Group H building blocks at the non-reducing end.

9. The sugar chain according to any one of claims 1 to 8, **characterized in that** the sugar chain is any one of Fucα1-2Galβ1-4(Fucα1-3)GlcNAcβ1-3Galβ1-3GalNAcα, Fucα1-2Galβ1-4(Fucα1-3)GlcNAcβ1-3GalNAcα, Neu5Acα2-3Galβ1-4GlcNAcβ1-3GalNAcα, Neu5Acα2-6Galβ1-4GlcNAcβ1-3(Neu5Acα2-6Galβ1-3GlcNAcβ1-6)GalNAcα, Galβ1-4(Fucα1-3)GlcNAcβ1-3Galβ1-3GalNAcα, Galβ1-4(Fucα1-3)GlcNAβ1-3GalNAcα, Fucα1-2Galβ1-4(Fucα1-3)GlcNAcβ1-6GalNAcα, 3 '-sialyllactose, 3 '-sialyllactosamine, 6'-sialyllactose, 6'-*N*-acetyl sialyllactosamine, Galβ1-3(Fucα1-4)GlcNAcβ1-3Galβ1-4Glc, Galβ1-4(Fucα1-3)GlcNAcβ1-3Galβ1-4Glc, Fucα1-2Galβ1-4GlcNAcβ1-3Galβ1-4Glc, Fucα1-2Galβ1-4(Fucα1-3)GlcNAcβ1-3Galβ1-4Glc, Galβ1-4(Fucα1-3)GlcNAcβ1-6(GlcNAcβ1-3)Galβ1-4Glc, Galβ1-4(Fucα1-3)GlcNAcβ1-6(Galβ1-4GlcNAcβ1-3)Galβ1-4Glc, Fucα1-2Galβ1-4GlcNAcβ1-6(Fucα1-2Galβ1-4GlcNAcβ1-3)Galβ1-4Glc, and Fucα1-2Galβ1-4(Fucα1-3)GlcNAcβ1-6(Fucα1-2Galβ1-4(Fucα1-3)GlcNAcβ1-3)Galβ1-4Glc.

10. A complex sugar chain, **characterized in that** the complex sugar chain is a sugar chain composition containing at least two different sugar chains, and at least one sugar chain in the sugar chain composition is the sugar chain according to any one of claims 1 to 9.

11. A complex sugar chain, **characterized in that** the complex sugar chain is a sugar chain composition containing at least two different sugar chains, and at least one sugar chain in the sugar chain composition contains any one or more of the following building blocks at the non-reducing end:
a) Neu5Acα2-6Gal;
b) Neu5Acα2-3Gal;
c) Lewis a;
d) Lewis x;
e) Lewis y; and
f) Blood Group H.

12. The complex sugar chain according to claim 10 or 11, **characterized in that** at least one sugar chain in the sugar chain composition is any one of sulfated fucoidan, HMOs, ginsenosides, chitosan oligosaccharide, heparin sulfate, heparin sulfate oligosaccharide, chondroitin sulfate, chondroitin sulfate oligosaccharide, dermatan sulfate, keratan sulfate, hyaluronic acid, hyaluronic acid oligosaccharide, fucoidan, fucose oligosaccharide, protein N/O-sugar chains, and exopolysaccharide from *Haloarcula hispanica.*

13. The complex sugar chain according to any one of claims 10 to 12, **characterized in that** at least one sugar chain in the sugar chain composition is any one of GlcNAcβ1-6(Galβ1-3)GalNAcα, GlcNAcβ1-6[Galβ1-4(Fucα1-3)GlcNAcβ1-3]Galβ1-4Glc, GlcNAcβ1-6[Fucα1-2Galβ1-4(Fucα1-3)GlcNAcβ1-3]Galβ1-4Glc, Galβ1-4GlcNAcβ1-6(Fucα1-2Galβ1-4GlcNAcβ1-3)Galβ1-4Glc, Galβ1-4(Fucα1-3)Glc, Fucα1-2Galβ1-4Glc, Galα1-3(Fucα1-2)Galβ1-4GlcNAcβ1-3NAcα, GlcNAcβ1-6(GlcNAcβ1-3)GalNAcα, and Galβ1-4(Fucα1-6)GlcNAcβ1-3GalNAcα,GlcNAcβ1-6(GlcNAcβ1-3)GalNAcα.

14. The complex sugar chain according to claim 10 or 11, **characterized in that** each sugar chain in the sugar chain composition contains any one or more of the following building blocks at the non-reducing end:
a) Neu5Acα2-6Gal;
b) Neu5Acα2-3Gal;
c) Lewis a;
d) Lewis x;
e) Lewis y; and
f) Blood Group H.

15. The complex sugar chain according to any one of claims 10 to 13, **characterized in that** at least one sugar chain in the sugar chain composition is exopolysaccharide from *Haloarcula hispanica.*

16. The complex sugar chain according to any one of claims 10 to 15, **characterized in that** the sugar chain composition contains at least one of Fucα1-2Galβ1-4(Fucα1-3)GlcNAcβ1-3Galβ1-3GalNAcα, and/or Fucα1-2Galβ1-4(Fucα1-3)GlcNAcβ1-3GalNAcα, and/or Neu5Acα2-3Galβ1-4GlcNAcβ1-3GalNAcα, and/or Neu5Acα2-6Galβ1-4GlcNAcβ1-3(Neu5Acα2-6Galβ1-3GlcNAcβ1-6)GalNAcα, and/or Galβ1-4(Fucα1-3)GlcNAcβ1-3Galβ1-3GalNAcα, and/or Galβ1-4(Fucα1-3)GlcNAcβ1-3GalNAcα, and/or Fucα1-2Galβ1-4(Fucα1-3)GlcNAcβ1-6GalNAcα, and/or 3'-sialyllactose, and/or 3'-sialyllactosamine, and/or 6'-sialyllactose, and/or 6'-*N*-acetyl sialyllactosamine, and/or Galβ1-3(Fucα1-4)GlcNAcβ1-3Galβ1-4Glc, and/or Galβ1-4(Fucα1-3)GlcNAcβ1-3Galβ1-4Glc, and/or Fucα1-2Galβ1-4GlcNAcβ1-3Galβ1-4Glc, and/or Fucα1-2Galβ1-4(Fucα1-3)GlcNAcβ1-3Galβ1-4Glc, and/or Galβ1-4(Fucα1-3)GlcNAcβ1-6(GlcNAcβ1-3)Galβ1-4Glc, and/or Galβ1-4(Fucα1-3)GlcNAcβ1-6(Galβ1-4GlcNAcβ1-3)Galβ1-4Glc, and/or Fucα1-2Galβ1-4GlcNAcβ1-6(Fucα1-2Galβ1-4GlcNAcβ1-3)Galβ1-4Glc, and/or Fucα1-2Galβ1-4(Fucα1-3)GlcNAcβ1-6(Fucα1-2Galβ1-4(Fucα1-3)GlcNAcβ1-3)Galβ1-4Glc.

17. Use of the sugar chain according to any one of claims 1 to 9 or the complex sugar chain according to any one of claims 10 to 16 in the preparation of a composition for preventing and/or treating coronavirus infection.

18. The use according to claim 17, **characterized in that** the coronaviruses comprise coronaviruses containing the S1 subunit, and/or coronaviruses capable of binding to the DC-SIGN receptor.

19. The use according to claim 17 or 18, **characterized in that** the viruses comprise 2019-nCov, MERS-CoV, SARS, and SARS-like viruses.

20. The use according to any one of claims 17 to 19, **characterized in that** the composition is used for topical administration.

21. The use according to any one of claims 17 to 20, **characterized in that** the composition is used for mucosal administration.

22. The use according to any one of claims 17 to 19, **characterized in that** the administration methods comprise any one of oral gargle, nasal rinse, and gasifying agent-assisted delivery to the lung.

23. Use of exopolysaccharide from *Haloarcula hispanica* in the preparation of a composition for preventing and/or treating coronavirus infection.

24. A composition for preventing and/or treating coronavirus infection, **characterized in that** an active ingredient of the pharmaceutical composition is the sugar chain according to any one of claims 1 to 9 or the complex sugar chain according to any one of claims 10 to 16.

25. A drug for preventing and/or treating coronavirus infection that is prepared from the composition for preventing and/or treating coronavirus according to claim 24.

26. A daily product for preventing and/or treating coronavirus infection that is prepared from the composition for preventing and/or treating coronavirus according to claim 24.

27. The drug for preventing and/or treating coronavirus infection according to claim 25, **characterized in that** the drug is in any form selected from powder, ointment, paste, emulsion, gel, solution, patch, and inhalant.

28. The daily product for preventing and/or treating coronavirus infection according to claim 26, **characterized in that** the daily product is used in the form of mouthwash or oral spray.

29. The daily product for preventing and/or treating coronavirus infection according to claim 26 or 28, **characterized in that** the active concentration of a sugar chain is 0.01%-1% (W/V).

30. Use of the sugar chain according to any one of claims 1 to 9 or the complex sugar chain according to any one of claims 10 to 16 in the preparation of a saccharide chip for preventing and/or treating coronavirus infection.

31. A saccharide chip for preventing and/or treating coronavirus infection, **characterized by** comprising the sugar chain according to any one of claims 1 to 9 or the complex sugar chain according to any one of claims 10 to 16.
